# EUROPEAN PATENT APPLICATION

(11) **EP 3 690 057 A1**
(43) Date of publication of application: **05.08.2020**
(21) Application number: 18861086.9
(22) Date of filing: 27.09.2018
(51) Int. Cl.: C12P 19/32, C12N 1/21, C12N 9/00, C12N 9/10, C12N 9/12, C12N 1/19

(54) **METHOD FOR PRODUCING NICOTINAMIDE MONONUCLEOTIDE AND TRANSFORMANT USED IN SAID METHOD**

(30) Priority: 29.09.2017 JP 2017190028
(71) Applicant: Mitsubishi Chemical Corporation, Tokyo 100-8251 (JP)
(72) Inventor: AKIYAMA Takanori, Tokyo 100-8251 (JP)
(74) Representative: Hoffmann Eitle
(86) International application number: PCT/JP2018/036040
(87) International publication number: WO 2019/065876

(57) **Abstract**

An object of the present invention is to provide a method for producing nicotinamide mononucleotide (NMN) with excellent production efficiency. The method for producing NMN according to the present invention (the first aspect) comprises the step of bringing a transformant with enhanced expression of enzymes nicotinamide phosphoribosyltransferase (Nampt), phosphoribosyl pyrophosphate synthetase (Prs) and polyphosphate kinase (Ppk), a cell-free protein synthesis reaction solution having the three enzymes expressed, or a treated product thereof into contact with a mixture containing ribose-5-phosphate (R5P), nicotinamide (NAM), ATP and polyphosphate.

## Description

### Technical Field

The present invention relates to a method for producing a nucleic acid compound such as nicotinamide mononucleotide.

### Background Art

Nicotinamide mononucleotide (NMN) is a synthetic intermediate of nicotinamide adenine dinucleotide (NAD+). In recent years, it has been revealed that: NMN controls the activity of a longevity gene "sirtuin" through conversion to NAD+; and NMN administered to mice exhibits an antiaging effect. NMN has been further reported to be also effective for preventing diseases such as diabetes mellitus, Alzheimer's disease and heart failure, or improving symptoms. Such NMN is expected for purposes as a component for functional foods, medicaments, cosmetics and the like. Research and development are underway on efficient methods for producing NMN with the aim of improving productivity.

Patent Literature 1 describes a method for producing NMN, comprising isolating cells (yeast, bacterium, etc.) caused to overexpress nicotinamide phosphoribosyltransferase (Nampt), and culturing the cells in the presence of nicotinamide (NAM) (claims 1, 13, 15, etc.). Nampt is an enzyme that generates NMN from NAM and intracellularly biosynthesized phosphoribosyl pyrophosphate (PRPP). Patent Literature 1 also states that the cells are further caused to overexpress phosphoribosyl pyrophosphate synthetase (Prs) in order to promote the biosynthesis of PRPP (claim 3, etc.). Prs is an enzyme that generates PRPP and AMP from ribose-5-phosphate (R5P) and ATP. In Patent Literature 1, the compound of interest such as NMN is generated through enzymatic reaction *in vivo* while cells overexpressing a particular enzyme are cultured in the living state in a medium containing a carbon source, a nitrogen source or the like.

Patent Literature 2 describes a NAD precursor synthesis system using a Prs mutant with attenuated sensitivity to reaction products (i.e., the amount of PRPP supplied into the system can be increased because the system is less likely to undergo a negative feedback by PRPP generated by Prs) (claim 1, paragraph 0068, etc.). The literature also states that this Prs mutant may be generated in a recombinant, isolated, and purified (claim 6, etc.). Patent Literature 2 also states that the system may further comprise Nampt, ATP, R5P and PRPP (claims 10, 20, 21, 23, etc.).

Meanwhile, Non Patent Literature 1 describes a method for synthesizing inosine monophosphate (IMP) from ribose using three enzymes ribokinase (Rbk; referred to as RK in the literature), Prs (referred to as PPS therein) and hypoxanthine phosphoribosyltransferase (8B3). Rbk is involved in (i) a reaction to generate R5P and ADP from ribose and ATP. Prs is involved in (ii) a reaction to generate PRPP and AMP from R5P and ATP. 8B3 is involved in (iii) a reaction to generate IMP and pyrophosphate (PPi) from PRPP and inosinic acid. The method described in Non Patent Literature 1 regenerates ADP through adenylate kinase from AMP generated in the reaction (ii). From the regenerated ADP and ADP generated in the reaction (i), ATP is regenerated through phosphoenolpyruvic acid and pyruvate kinase.

Patent Literature 3 describes a method for producing ATP, comprising reacting polyphosphate kinase (Ppk) type 2, which is an enzyme capable of synthesizing ATP from AMP by itself, with polyphosphate and AMP. Thermophile (*Thermosynechococcus elongatus,* etc.)-derived polyphosphate kinase having a particular amino acid sequence is disclosed as such polyphosphate kinase type 2. Patent Literature 3 further states that a method for producing a substance (compound of interest) through the use of ATP can be carried out at the same time with the method for producing ATP, thereby coupling the synthesis reaction of the compound of interest to ATP regeneration reaction, so that ATP regenerated from AMP is utilized in the synthesis of the compound of interest.

Patent Literature 4 describes a method comprising heating *Escherichia coli* containing a gene of thermophile-derived Ppk that is not inactivated even by heat treatment at 70°C for 10 min), at 60 to 80°C (at which general enzymes of *Escherichia coli* lose their activity, and the cell permeability of polyphosphate is obtained) to perform ATP regeneration reaction in a state where the cell membrane of *Escherichia coli* is disrupted.

Patent Literature 5 describes a method for producing nicotinamide mononucleotide, comprising using NAM, ATP and ribose as starting materials, and reacting the starting materials under the catalytic effects of nicotinamide phosphoribosyltransferase, ribose phosphate pyrophosphokinase and ribose kinase.

Non Patent Literature 2 describes a method for synthesizing glycerol triphosphate through the use of thermophile-derived Ppk and an ATP regeneration system based thereon. In the method, a buffer containing glycerol, ADP, polyphosphate, glycerol kinase (GK), Ppk and the like is used as an initial reaction solution, and the reaction is allowed to proceed while polyphosphate is successively added.

Non Patent Literature 3 describes a method for producing glutathione through cascade reaction in which *Streptococcus* sanguinis-derived glutathione synthase (GshF) is coupled to an ATP regeneration system based on thermophile *Thermosynechococcus elongates* BP-1-derived Ppk having the same activity as described in Patent Literature 3.

### Citation List

### Patent Literature

Patent Literature 1: WO 2015/069860
Patent Literature 2: WO 2016/198948
Patent Literature 3: JP 2013-021967 A
Patent Literature 4: JP 2007-143463 A
Patent Literature 5: WO 2017/185549

### Non Patent Literature

Non Patent Literature 1: Scism, Robert A. and Bachmann, Brian O. "Five-component cascade synthesis of nucleotide analogues in an engineered self-immobilized enzyme
   aggregate." ChemBioChem 11.1 (2010): 67-70.
Non Patent Literature 2: Restiawaty, Elvi et al. "Feasibility of thermophilic adenosine triphosphate-regeneration system using Thermus thermophilus polyphosphate kinase." Process Biochemistry 46.9 (2011): 1747-1752.
Non Patent Literature 3: Zhang, Xing et al. "One-pot synthesis of glutathione by a two-enzyme cascade using a thermophilic ATP regeneration system." Journal of Biotechnology 241 (2017): 163-169.

### Summary of Invention

### Technical Problem

However, the methods disclosed in Patent Literatures 1 to 5 and Non Patent Literatures 1 to 3 have problems associated with efficient production of NMN and cannot be excellent methods. Specifically, in Patent Literature 1, NMN is generated through enzymatic reaction *in vivo* while cells overexpressing a particular enzyme are cultured in the living state in a medium containing a carbon source, a nitrogen source or the like. The amount of NMN generated is 300 nmol-NMN/g-yeast wet cell weight (Example 1). If the yeast dry cell weight/yeast wet cell weight ratio is 0.2, the amount of NMN generated is disadvantageously as low as approximately 0.5 g-NMN/kg-yeast dry cell weight.

Patent Literature 2 describes a NAD precursor synthesis system using a Prs mutant with attenuated sensitivity to reaction products. However, the literature describes no Example showing the generation of NMN, and discloses neither a specific method for producing NMN nor amount of NMN generated.

Patent Literature 3 describes a method for producing a substance, wherein a method for producing ATP by reacting polyphosphate kinase (Ppk) type 2, which is an enzyme capable of synthesizing ATP from AMP by itself, with polyphosphate and AMP is coupled to the synthesis reaction of the compound of interest. However, the literature discloses neither a specific method for producing NMN nor amount of NMN generated.

Patent Literature 4 describes a method comprising heating *Escherichia coli* containing a gene of thermophile-derived Ppk, and performing ATP regeneration reaction in a state where the cell membrane of *Escherichia coli* is disrupted. However, the literature discloses neither a specific method for producing NMN nor amount of NMN generated.

Patent Literature 5 describes a method for producing nicotinamide mononucleotide, comprising using nicotinamide, ATP and ribose as starting materials, and reacting the starting materials under the catalytic effects of nicotinamide phosphoribosyltransferase, ribose phosphate pyrophosphokinase and ribose kinase. However, in Patent Literature 5, a large amount of ATP is used in the production of NMN. Specifically, for the NMN production, the number of moles of ATP to be added to the reaction system is two or more times the number of moles of NMN to be generated. Since ATP is an expensive starting material, the production method cannot have cost efficiency.

Accordingly, many methods for producing NMN have been disclosed so far, but are not sufficient from the viewpoint of the amount of NMN generated and the amount of ATP necessary for producing NMN.

An object of the present invention is to provide a method for producing nicotinamide mononucleotide with excellent production efficiency.

### Solution to Problem

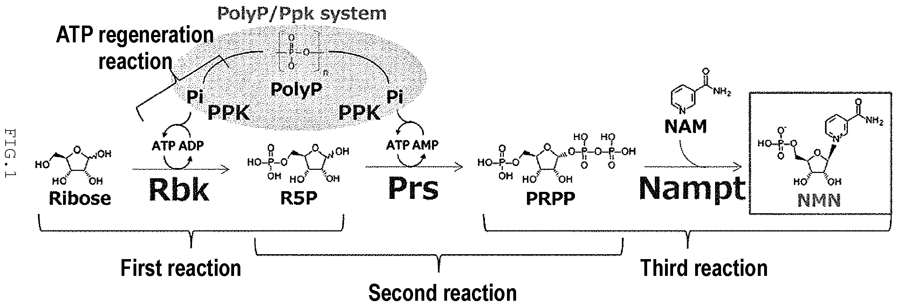

The present inventors have found that in the case of allowing enzymatic reaction to proceed by bringing a transformant with enhanced expression of three enzymes Nampt, Prs and Ppk, a cell-free protein synthesis reaction solution having the three enzymes expressed, or a treated product thereof into contact with R5P, NAM, ATP and polyphosphate (see Figure 1), NMN can be produced more efficiently and inexpensively than conventional methods. The present inventors have further found that in the case of producing the R5P by bringing a transformant with enhanced expression of two enzymes Rbk and Ppk, a cell-free protein synthesis reaction solution having the two enzymes expressed, or a treated product thereof into contact with ribose, ATP and polyphosphate, NMN can be produced much more efficiently and inexpensively. These findings have led to the completion of the first aspect.

The present inventors have also found that in the case of allowing enzymatic reaction to proceed by bringing a transformant with enhanced expression of Nampt, a cell-free protein synthesis reaction solution having the enzyme expressed, or a treated product thereof into contact with NAM and PRPP in the presence of pyrophosphatase (PPase), NMN can be produced more efficiently than conventional methods. These findings have led to the completion of the second aspect.

The present inventors have further found that NMN can be efficiently produced while the degradation of NMN is markedly suppressed, by bringing a transformant with enhanced expression of nicotinamide phosphoribosyltransferase (Nampt) or a treated product thereof into contact with at least nicotinamide (NAM), wherein the transformant has disruption or deletion of a gene encoding an enzyme classified into EC number represented by (d) EC 3.5.1.42, and a gene encoding an enzyme classified into at least any one of EC numbers represented by (a), (c), (g), (h) and (i) given below. These findings have led to the completion of the third aspect.
(a) EC 3.1.3.5
(c) EC 2.4.2.1
(g) EC 3.2.2.1
(h) EC 3.2.2.3
(i) EC 3.2.2.14

Moreover, the present inventors have found that by approaches used alone or in combination, the total sum of the respective numbers of moles of ATP, ADP and AMP to be added to the reaction system for NMN production can be 0.5 equivalents or less of the number of moles of NMN to be generated. These findings have led to the completion of the fourth aspect.

Specifically, the present invention relates to [item 1] to [item 17].
[Item 1]
   A method for producing nicotinamide mononucleotide (NMN), comprising the step of bringing a transformant with enhanced expression of three enzymes nicotinamide phosphoribosyltransferase (Nampt), phosphoribosyl pyrophosphate synthetase (Prs) and polyphosphate kinase (Ppk), a cell-free protein synthesis reaction solution having the three enzymes expressed, or a treated product thereof into contact with ribose-5-phosphate (R5P), nicotinamide (NAM), ATP and polyphosphate.
[Item 2]
   The method for producing NMN according to item 1, further comprising the step of bringing a transformant with enhanced expression of two enzymes ribokinase (Rbk) and polyphosphate kinase (Ppk), a cell-free protein synthesis reaction solution having the two enzymes expressed, or a treated product thereof into contact with ribose, ATP and polyphosphate to produce the R5P.
[Item 3]
   The method for producing NMN according to item 1 or 2, wherein the method is performed under conditions where the transformant does not substantially proliferate.
[Item 4]
   The method for producing NMN according to any one of items 1 to 3, wherein the Nampt is bacterium-derived.
[Item 5]
   The method for producing NMN according to any one of items 1 to 4, wherein the Ppk belongs to the polyphosphate kinase type 2 family.
[Item 6]
   The method for producing NMN according to any one of items 1 to 5, wherein the Ppk belongs to the class 3 subfamily of the polyphosphate kinase type 2 family (Ppk2 class 3).
[Item 7]
   The method for producing NMN according to any one of items 1 to 6, wherein a host of the transformant is *Escherichia coli,* a bacterium of the genus *Corynebacterium,* a bacterium of the genus *Rhodococcus,* or a yeast.
[Item 8]
   A transformant with enhanced expression of three enzymes nicotinamide phosphoribosyltransferase (Nampt), phosphoribosyl pyrophosphate synthetase (Prs) and polyphosphate kinase (Ppk).
[Item 9]
   A transformant with enhanced expression of two enzymes ribokinase (Rbk) and polyphosphate kinase (Ppk).
[Item 10]
   A method for producing nicotinamide mononucleotide (NMN), comprising the step of bringing a transformant with enhanced expression of nicotinamide phosphoribosyltransferase (Nampt), a cell-free protein synthesis reaction solution having the enzyme expressed, or a treated product thereof into contact with nicotinamide (NAM) and phosphoribosyl pyrophosphate (PRPP) in the presence of pyrophosphatase (PPase).
[Item 11]
   The method for producing NMN according to item 10, wherein the method is performed under conditions where the transformant does not substantially proliferate.
[Item 12]
   The method for producing NMN according to item 10 or 11, wherein the treated product is a purified enzyme.
[Item 13]
   The method for producing NMN according to any one of items 10 to 12, wherein the Nampt is bacterium-derived.
[Item 14]
   A method for producing NMN, comprising the step of bringing a transformant with enhanced expression of nicotinamide phosphoribosyltransferase (Nampt) or a treated product thereof into contact with at least nicotinamide (NAM), wherein the transformant has disruption or deletion of a gene encoding an enzyme classified into EC number represented by (d) EC 3.5.1.42, and a gene encoding an enzyme classified into at least any one of EC numbers represented by the following (a), (c), (g), (h) and (i) :
   (a) EC 3.1.3.5,
   (c) EC 2.4.2.1,
   (g) EC 3.2.2.1,
   (h) EC 3.2.2.3, and
   (i) EC 3.2.2.14.
[Item 15]
   The method for producing NMN according to any one of items 1 to 14, wherein the total sum of the respective numbers of moles of ATP, ADP and AMP to be added to the reaction system is 0.5 equivalents or less of the number of moles of NMN to be generated.
[Item 16]
   A method for producing nicotinamide mononucleotide (NMN), comprising the step of bringing a transformant with enhanced expression of two enzymes nicotinamide phosphoribosyltransferase (Nampt) and phosphoribosyl pyrophosphate synthetase (Prs), a cell-free protein synthesis reaction solution having the two enzymes expressed, or a treated product thereof into contact with ribose-5-phosphate (R5P), nicotinamide (NAM) and ATP, wherein the total sum of the respective numbers of moles of ATP, ADP and AMP to be added to the reaction system is 0.5 equivalents or less of the number of moles of NMN to be generated.
[Item 17]
   The method for producing NMN according to item 16, further comprising the step of bringing a transformant with enhanced expression of ribokinase (Rbk), a cell-free protein synthesis reaction solution having the enzyme expressed, or a treated product thereof into contact with ribose and ATP to produce the R5P.

### Advantageous Effects of Invention

Use of the method for producing NMN according to the first aspect of the present invention can drastically improve the amount of NMN produced as compared with conventional methods (e.g., to 10 times the amount of NMN produced described in Patent Literature 1). The production method of the present invention utilizing ATP regeneration reaction can produce NMN by using more inexpensive R5P or ribose as a starting material without adding a large amount of an expensive intermediate such as PRPP, or ATP, and is therefore capable of reducing production cost.

Use of the method for producing NMN according to the second aspect of the present invention can allow the third reaction to proceed efficiently. This permits improvement in the amount of NMN produced and a NMN generation rate and can reduce NMN production cost.

Use of the method for producing NMN according to the third aspect of the present invention can suppress the degradation of a product NMN in performing NMN generation reaction using a transformant, dormant cells, cells with improved membrane permeability, inactivated cells and homogenized cells prepared from the transformant, cell-free extracts prepared from the homogenized cells, and stabilized products obtained by stabilization treatment thereof. This enables NMN to be produced at high yields using microbial cells, cell-free extracts or the like in an inexpensive catalytic form, and can reduce NMN production cost.

Use of the method for producing NMN according to the fourth aspect of the present invention can reduce the amount of ATP added for NMN production. Since ATP is expensive, this can reduce NMN production cost.

### Brief Description of Drawings

[Figure 1] Figure 1 is a schematic diagram showing reactions that proceed in the method for producing NMN according to the present invention.
[Figure 2] Figure 2 is a graph showing a NMN generation concentration in Example 1 and Comparative Example 2.
[Figure 3] Figure 3 is a graph showing a NMN generation concentration in Example 3.
[Figure 4] Figure 4 is a graph showing a NMN generation concentration in Example 4.
[Figure 5] Figure 5 is a graph showing a NMN generation concentration in Example 5.
[Figure 6] Figure 6 is a graph showing a NMN generation concentration in Example 6 and Comparative Example 2.
[Figure 7] Figure 7 is a graph showing a NMN generation concentration in Example 7.
[Figure 8] Figure 8 is a graph showing a NMN generation concentration in Example 8.
[Figure 9] Figure 9 is a diagram showing NMN degradation pathways.

### Description of Embodiments

Abbreviations used in the present specification are each defined as follows.
Nampt: nicotinamide phosphoribosyltransferase
Prs: phosphoribosyl pyrophosphate synthetase
Rbk: ribokinase
Ppk: polyphosphate kinase
PPase: pyrophosphatase
NMN: nicotinamide mononucleotide
PRPP: phosphoribosyl pyrophosphate
NAM: nicotinamide
R5P: ribose-5-phosphate
NR: nicotinamide riboside
NaMN: nicotinic acid mononucleotide
NAD: nicotinamide adenine dinucleotide
PPi: pyrophosphate
PolyP: polyphosphate

### - First aspect -

The method for producing NMN according to the first aspect of the present invention comprises the step of bringing a transformant with enhanced expression of three enzymes Nampt, Prs and Ppk, a cell-free protein synthesis reaction solution having the three enzymes expressed, or a treated product thereof into contact with R5P, NAM, ATP and polyphosphate. Preferably, the method for producing NMN according to the present invention further comprises, as a step for producing the R5P, the step of bringing a transformant with enhanced expression of two enzymes Rbk and Ppk, a cell-free protein synthesis reaction solution having the two enzymes expressed, or a treated product thereof into contact with a mixture containing ribose, ATP and polyphosphate. Specifically, in the present invention, NMN is produced by allowing predetermined enzymatic reaction to proceed while utilizing ATP regeneration reaction.

Such a method for producing NMN according to the first aspect is typically carried out by performing steps (1) to (3) given below (in the present specification, referred to as the first step, the second step and the third step, respectively) in order. These steps may be carried out by one person or may be carried out by different persons. These steps may be continuously performed or may be performed in stages at predetermined intervals between the steps.
(1) The step of preparing and culturing a transformant containing a gene encoding each of enzymes Nampt, Prs, Rbk and Ppk, or performing protein synthesis reaction in a cell-free protein synthesis reaction solution containing a gene encoding each of the enzymes to express the enzymes (first step);
(2) the step of preparing, if necessary, a treated product from the transformant or the cell-free protein synthesis reaction solution after the first step (second step); and
(3) the step of bringing the transformant, the cell-free protein synthesis reaction solution, or the treated product thereof after the first and second steps into contact with each substrate compound (third step; see Figure 1).

Hereinafter, the method for producing NMN according to the present invention will be described in more detail with reference to an embodiment in which the first step, the second step and the third step are performed. However, the method for producing NMN according to the present invention may be performed by embodiments in which the first step, the second step and the third step specifically described below are appropriately changed or modified without departing from the spirit of the present invention.

### [First step]

The first step is the step of preparing and culturing a transformant containing a gene encoding each of enzymes Nampt, Prs, Rbk and Ppk, or performing protein synthesis reaction in a cell-free protein synthesis reaction solution containing a gene encoding each of the enzymes to express the enzymes.

### (Enzyme)

In the present invention, four enzymes, i.e., Nampt, Prs and Ppk and further Rbk, if necessary, are utilized. All of Nampt, Prs, Ppk and Rbk are known enzymes, and their amino acid sequences and nucleotide sequences of genes encoding these enzymes are readily available to those skilled in the art. These predetermined four enzymes may be natural enzymes or may be mutant enzymes, preferably with improved expression level or enzyme activity, prepared by modifying the amino acid sequences of the natural enzymes as long as the enzymes can catalyze their respective reactions of interest. Any of various tags (proteins or peptides) may be added to each enzyme for the purpose of, for example, simplifying purification, promoting the exertion of solubility, or detecting the enzyme with an antibody. Examples of the types of the tags include His tag (histidine tag), Strep (II)-tag, GST tag (glutathione-S-transferase tag), MBP tag (maltose binding protein tag), GFP tag (green fluorescent protein tag), SUMO tag (small ubiquitin-related (like) modifier tag), FLAG tag, HA tag and myc tag. The four enzymes may be mutually expressed as a fusion protein.

### Nampt

Nampt (EC number: 2.4.2.12) is generally known to participate in the NAD (nicotinamide adenine dinucleotide) salvage pathway. In the present invention, this enzyme is utilized for a reaction to generate NMN from PRPP and NAM (third reaction). ATP is not originally essential for the Nampt-mediated NMN synthesis reaction from PRPP and NAM. However, Nampt has ATP hydrolytic activity, and enzymatic parameters or chemical equilibrium has been reported to shift in favor of NMN generation by the autophosphorylation of Nampt through the hydrolysis of ATP (Biochemistry 2008, 47, 11086-11096).

Examples of Nampt include an enzyme derived from humans (*Homo sapiens*) (NP_005737), an enzyme derived from mice (*Mus musculus*) (NP_067499), an enzyme derived from rats (*Rattus norvegicus*) (NP_808789), an enzyme derived from zebra fishes (*Danio rerio*) (NP_997833), and enzymes derived from bacteria such as *Haemophilus ducreyi* (AAR87771), *Deinococcus radiodurans* (AE001890), *Oenococcus oeni* (KZD13878) and *Shewanella oneidensis* (NP_717588). In the present invention, bacterium-derived Nampt is preferably used because of being excellent in enzyme activity in the third reaction. In this context, the bacteria are a group of prokaryotes having no nuclear membrane and are an organism group including *Escherichia coli, Bacillus subtilis,* cyanobacteria and the like.

### Prs

In the present invention, Prs (EC number: 2.4.2.17) is an enzyme that is utilized for a reaction to generate PRPP and AMP from R5P and ATP (second reaction).

Examples of Prs include an enzyme derived from humans (*Homo sapiens*) (NP_002755), a *Bacillus subtilis-*derived enzyme (BAA05286), a *Bacillus caldolyticus-*derived enzyme (CAA58682), an *Arabidopsis thaliana-*derived enzyme (Q680A5) and a *Methanocaldococcus jannaschii*-derived enzyme (Q58761). Particularly, when the substrate concentration in the production system is high over a long time, mutant Prs as described in Patent Literature 2 may be used such that the generation of PRPP by the second reaction and the generation of NMN by the third reaction subsequent thereto can be continued (i.e., the concentration of the final product NMN in the production system can keep increasing). Examples of the mutant Prs include mutants of human-derived Prs, such as Asp51His (substitution of ASP at position 51 by His; the same holds true for the description below), Asn113Ser, Leu128Ile, Asp182His, Ala189Val and His192Gln, and mutants of Prs derived from other organisms corresponding thereto, for example, mutants of *Bacillus subtilis-*derived Prs, such as Asn120Ser (which corresponds to Asn113Ser described above) and Leu135Ile (which corresponds to Leu128Ile described above).

### Rbk

In the present invention, Rbk (EC number: 2.7.1.15) is an enzyme that is utilized for a reaction to generate R5P and ADP from ribose and ATP (first reaction). Natural Rbk derived from various organisms, or mutant Rbk prepared by modifying the amino acid sequence of the natural Rbk can be used as Rbk. Examples thereof include an enzyme derived from humans (*Homo sapiens*) (NP_002755), an enzyme derived from yeasts (*Saccharomyces cerevisiae*) (P25332), a *Bacillus* subtilis-derived enzyme (P36945), an *Escherichia* coli-derived enzyme (AAA51476) and a *Haemophilus influenzae-derived* enzyme (P44331).

### Ppk

In the present invention, Ppk (EC number: 2.7.4.1) is an enzyme that is utilized for a reaction to regenerate ATP from ADP generated by the first reaction or AMP generated by the second reaction, and polyphosphate (ATP regeneration reaction).

Ppk can be classified into two families, the polyphosphate kinase type 1 family (Ppk1) and the polyphosphate kinase type 2 family (Ppk2), according to difference in amino acid sequence and kinetics. The activity of regenerating ATP with polyphosphate as a substrate is higher in Ppk2 than in Ppk1. Thus, in the present invention, Ppk2 is preferably used as Ppk.

Ppk2 can be further classified into three subfamilies, class 1, class 2 and class 3. Ppk2 class 1 and Ppk2 class 2 catalyze a reaction to generate ATP by phosphorylating ADP, and a reaction to generate ADP by phosphorylating AMP, respectively. By contrast, Ppk2 class 3 can catalyze both the AMP phosphorylation reaction and the ADP phosphorylation reaction and can therefore generate ATP from AMP by itself.

In the present invention, Ppk2 class 1 for regenerating ATP from ADP and Ppk2 class 2 for regenerating ADP from AMP can be used in combination as Ppk. Alternatively, in the case of using Ppk in combination with adenylate kinase (which catalyzes a reaction of AMP + ATP → 2ADP) for regenerating ADP from AMP, only Ppk2 class 1 or Ppk1 for regenerating ATP from ADP may be used as Ppk in the present invention. However, Ppk2 class 3 is preferably used because of its favorable efficiency with which the enzyme can catalyze both the reactions to regenerate ATP from ADP and to regenerate ATP from AMP by itself. Use of such Ppk can achieve the commonality of Ppk for the first reaction and Ppk for the second reaction.

Examples of Ppk2 class 3 include a *Deinococcus radiodurans-derived* enzyme (NP_293858), a *Paenarthrobacter* aurescens-derived enzyme (ABM08865), a *Meiothermus* ruber-derived enzyme (ADD29239), a *Deinococcus* geothermalis-derived enzyme (WP_011531362) and a *Thermosynechococcus* elongatus-derived enzyme (NP_682498). On the other hand, examples of Ppk2 class 1 include a *Rhodobacter* sphaeroides-derived enzyme (CS253628), a *Sinorhizobium meliloti-derived* enzyme (NP_384613), *Pseudomonas aeruginosa-derived* PA0141 (NP_248831), *Pseudomonas aeruginosa-derived* PA2428 (NP_251118), and a *Francisella* tularensis-derived enzyme (AJI69883). Examples of Ppk2 class 2 include *Pseudomonas aeruginosa-derived* PA3455 (NP_252145). Examples of the adenylate kinase include a *Bacillus* cereus-derived enzyme (AAP07232).

### (Transformant)

The transformant used in the present invention exhibits enhanced expression of each predetermined enzyme as compared with an untransformed (wild-type) cell or microbial cell. The "enhanced expression" does not generalize the degree to which the expression level of each enzyme is enhanced. As mentioned later, the expression level in the transformant can be adjusted such that the concentration of each enzyme in a reaction solvent (in the production system) falls within an appropriate range. The expression can be enhanced at least as compared with an untransformed (wild-type) cell or microbial cell by an artificial operation. Examples of the artificial operation include, but are not particularly limited to, an operation of utilizing an expression vector as mentioned below, an operation of introducing multiple copies of a gene expression unit encoding a predetermined enzyme onto the genome, and an operation of replacing an original promoter of a gene encoding a predetermined enzyme on the genome with a strong one.

The transformant used in the present invention may consist of (i) a transformant alone containing all of genes encoding predetermined enzymes, or may be constituted by, for example, (ii) a combination consisting of a transformant with enhanced expression of Nampt and Prs and a transformant with enhanced expression of Ppk, as a combination of transformants separately containing genes encoding predetermined enzymes.

The host of the transformant is not particularly limited as long as the cells can express a predetermined enzyme by a protein expression system through the use of an expression vector or the like. Examples thereof include: bacteria such as *Escherichia coli, Bacillus subtilis,* and actinomycetes (e.g., the genus *Rhodococcus* and the genus *Corynebacterium);* yeasts (e.g., the genus *Saccharomyces,* the genus *Candida* and the genus *Pichia*); filamentous fungi; plant cells; and animal cells such as insect cells and mammalian cells. Among them, *Escherichia coli,* a bacterium of the genus *Corynebacterium* and a bacterium of the genus *Rhodococcus* as well as a yeast of the genus *Saccharomyces,* a yeast of the genus *Candida* and a yeast of the genus *Pichia* are preferred, and *Escherichia coli* is more preferred.

Examples of the *Escherichia coli* include *Escherichia coli* K12 and B strains as well as derivatives derived from these wild strains, such as W3110, JM109, XL1-Blue (e.g., XL1-Blue MRF'), K802, C600, BL21 and BL21 (DE3) strains.

In the case of using an expression vector, the expression vector is not particularly limited as long as a gene of each predetermined enzyme used in the present invention is contained therein in an expressible state. A vector suitable for each host can be used. The detailed configuration of the expression vector will be mentioned later.

The method for transfecting the host with the expression vector is not particularly limited as long as the method is suitable for the host. Examples of the method that may be utilized include electroporation, a method using calcium ions, a spheroplast method, a lithium acetate method, a calcium phosphate method and lipofection.

The transformant thus harboring the expression vector can be cultured by a method suitable for the cells (microbial cells) used as the host to express each predetermined enzyme. In the case of combining transformants separately containing genes encoding predetermined enzymes as mentioned above, for example, in the case of preparing and combining a transformant with enhanced expression of Nampt and Prs and a transformant with enhanced expression of Ppk, these transformants may be cultured in the same medium or may be cultured in separate media and then mixed.

In the case of performing NMN generation reaction using the transformant, dormant cells, cells with improved membrane permeability, inactivated cells or homogenized cells prepared from the transformant, cell-free extracts prepared from the homogenized cells, or a stabilized product (for the details, see items related to the second step described later) obtained by stabilization treatment thereof, NMN may be unable to be efficiently produced because the degradation or side reaction of the reactant (substrate) ribose or NAM or the product NMN occurs. In this case, a host can be used which has disruption or deletion of a gene responsible for the degradation or the side reaction. Specifically, a host can be used which has disruption or deletion of a gene encoding an enzyme classified into at least any one of EC numbers represented by the following (a) to (i).
(a) EC 3.1.3.5
(b) EC 3.5.1.19
(c) EC 2.4.2.1
(d) EC 3.5.1.42
(e) EC 1.17.2.1
(f) EC 1.17.1.5
(g) EC 3.2.2.1
(h) EC 3.2.2.3
(i) EC 3.2.2.14

The enzyme classified into (a) EC 3.1.3.5 is 5'-nucleotidase and includes an enzyme that catalyzes a reaction to generate nicotinamide riboside (NR) and phosphate by hydrolyzing NMN. Examples of the gene encoding the 5'-nucleotidase include *Escherichia coli* ushA, surE, yrfG and yjjG. For example, Enzyme and Microbial Technology, 58-59 (2014), 75-79 has reported 5'-nucleotidase UshA as an enzyme that plays a key role in degrading NAD in *Escherichia coli,* and discloses that the enzyme has NMN degradative activity. In the present invention, the 5'-nucleotidase to be disrupted or deleted is particularly preferably ushA or a homolog gene thereof.

The enzyme classified into (b) EC 3.5.1.19 is nicotinamidase and is involved in the degradation of NAM. Examples of the gene encoding the nicotinamidase include *Escherichia coli* pncA.

The enzyme classified into (c) EC 2.4.2.1 is purine-nucleoside phosphorylase and includes an enzyme that catalyzes a reaction to generate NAM and ribose-1-phosphate (R1P) by the phosphorolysis of NR. Examples of the gene encoding the purine-nucleoside phosphorylase include *Escherichia coli* deoD. Molecular and General Genetics, 104 (1969), 351-359 discloses deoD as a member of a gene group involved in nucleoside metabolism. In the present invention, the purine-nucleoside phosphorylase gene to be disrupted or deleted is preferably deoD or a homolog gene thereof.

The enzyme classified into (d) EC 3.5.1.42 is nicotinamide mononucleotide deamidase and is an enzyme that generates NaMN and ammonia by hydrolyzing NMN. Examples of the gene encoding the nicotinamide mononucleotide deamidase include *Escherichia coli* pncC. THE JOURNAL OF BIOLOGICAL CHEMISTRY, 286 (2011), 40365-40375 discloses findings about *Shewanella oneidensis* and *Escherichia coli* pncC. In the present invention, the nicotinamide mononucleotide deamidase to be disrupted or deleted is preferably pncC or a homolog gene thereof.

Each of the enzymes classified into (e) EC 1.17.2.1 and (f) EC 1.17.1.5 is nicotinate dehydrogenase and is considered to possibly participate in the production of hydroxynicotinate as a by-product from NAM.

The enzyme classified into (g) EC 3.2.2.1 is purine nucleosidase and includes an enzyme that catalyzes a reaction to generate NAM and ribose by hydrolyzing NR. Examples of the gene encoding the purine nucleosidase include *Ochrobactrum anthropi* Pu-N (Applied and Environmental Microbiology, 67 (2001), 1783-1787). In the present invention, the purine nucleosidase to be disrupted or deleted is preferably Pu-N or a homolog gene thereof.

The enzyme classified into (h) EC 3.2.2.3 is uridine nucleosidase and includes an enzyme capable of catalyzing a reaction to generate NAM and ribose by hydrolyzing NR. Examples of the gene encoding the uridine nucleosidase include *Arabidopsis thaliana* urh1 (Plant Cell, 21 (2009), 876-91). In the present invention, the uridine nucleosidase to be disrupted or deleted is preferably urh1 or a homolog gene thereof.

The enzyme classified into (i) EC 3.2.2.14 is NMN nucleosidase and is an enzyme that catalyzes a reaction to generate NAM and R5P by hydrolyzing NMN. Biochem. Biophys. Res. Commun., 49 (1972), 264-9 discloses NMN nucleosidase activity in *Escherichia coli.* In the present invention, a gene encoding the enzyme having the NMN nucleosidase activity is preferably disrupted or deleted.

The gene to be deleted or disrupted is preferably a gene encoding an enzyme classified into EC number represented by (d) and a gene encoding an enzyme classified into at least any one of EC numbers represented by (a), (c), (g), (h) and (i), more preferably a gene encoding an enzyme classified into EC number represented by (d) and a gene encoding an enzyme classified into EC number represented by (a). The details will be described in the description about the third aspect mentioned later.

The method for disrupting or deleting each gene is not particularly limited and can be performed by a gene disruption or deletion method known in the art. Examples thereof include a method using a linearized fragment for gene disruption or deletion, a method using a circular gene disruption or deletion plasmid containing no replication origin, a method using group II introns, a Red-ET homologous recombination method, and a method using genome editing with ZFN, TALEN, CRISPR/Cas9 or the like.

### (Expression vector)

The gene encoding each predetermined enzyme used in the present invention is typically transferred in a state contained in an expression vector to the host of the transformant. In the case of expressing two or more enzymes in one transformant, all the genes encoding the enzymes to be expressed may be contained in one expression vector, or the genes encoding the enzymes to be expressed may be appropriately assigned to and contained in two or more expression vectors capable of coexisting in the host. In the case of preparing, for example, a transformant with enhanced expression of Nampt, Prs and Ppk, one expression vector containing all the genes encoding Nampt, Prs and Ppk may be used, or two vectors, i.e., an expression vector containing genes encoding Nampt and Prs and an expression vector containing a gene encoding Ppk, may be configured in combination.

The expression vector used in the present invention can be prepared by an approach known in the art. In general, a transcription promoter is inserted to upstream of a gene encoding each predetermined enzyme, and optionally, a terminator is inserted to downstream of the gene to construct an expression cassette. This cassette can be inserted to an expression vector. Alternatively, when the expression vector already contains a transcription promoter and/or a terminator, the gene encoding the predetermined enzyme can be inserted to between the transcription promoter and/or the terminator of the vector without constructing an expression cassette. As mentioned above, when genes encoding two or more enzymes are contained in one expression vector, all the genes may be inserted under the same promoter or may be inserted under different promoters. The type of the promoter is not particularly limited as long as the promoter permits appropriate expression in the host. Examples of the promoter that can be utilized in an *Escherichia coli* host include T7 promoter, trp promoter, lac promoter, lambda phage-derived PL promoter and PR promoter, tac promoter, and trc promoter.

The gene (nucleic acid) encoding each predetermined enzyme may be obtained, for example, by (i) preparing primers according to nucleotide sequence information, followed by amplification using a template such as the genome, or (ii) synthesizing DNA in an organic synthetic manner according to amino acid sequence information on the enzyme. The gene may be optimized for the cells serving as the host of the transformant.

For example, a method using restriction enzymes, or a method using topoisomerase can be utilized for inserting the gene encoding each predetermined enzyme to the expression vector. For the insertion, an appropriate linker may be added, if necessary. A ribosome binding sequence such as a SD sequence or a Kozak sequence is known as a nucleotide sequence important for translation into amino acids. Such a sequence may be inserted to upstream of the gene. The amino acid sequence encoded by the gene may be partially substituted along with the insertion. The vector preferably contains a factor for screening for the transformant of interest (selective marker). Examples of the selective marker include drug resistance genes, auxotrophic complementary genes and assimilability-imparting genes. The selective marker can be selected according to a purpose or the host. Examples of the drug resistance gene for use as the selective marker in *Escherichia coli* include ampicillin resistance gene, kanamycin gene, dihydrofolate reductase gene and neomycin resistance gene.

An expression vector appropriately selected from plasmid DNA, bacteriophage DNA, retrotransposon DNA, artificial chromosomal DNA and the like according to the host can be used. In the case of using, for example, *Escherichia coli* as the host, examples thereof can include pTrc99A (GE Healthcare Bio-Sciences Corp.), pACYC184 (Nippon Gene Co., Ltd.), pMW118 (Nippon Gene Co., Ltd.) and pET series vectors (Novagen). Examples of the vector having two or more insertion locations can include pETDuet-1 (Novagen). Modified forms of these vectors may be used, if necessary.

The method for enhancing the expression of each predetermined enzyme is typically a method using the expression vector as mentioned above. However, other methods can also be utilized. The expression of the predetermined enzyme can be enhanced, for example, by inserting an expression cassette in which a gene encoding the predetermined enzyme is linked to an appropriate promoter, terminator, marker gene or the like onto the genome of the host. A method known in the art can be used as a method for obtaining a transformant having an expression cassette inserted on the genome. In the case of inserting an expression cassette onto the genome, for example, by homologous recombination, the host can be transformed with a plasmid that has an expression cassette for the predetermined enzyme and the sequence of an arbitrary genome region and is incapable of replicating in the host, to obtain a transformant having an insert of the whole plasmid or the expression cassette. In this respect, a transformant having only the expression cassette inserted on the genome may be efficiently obtained by two rounds of homologous recombination using a plasmid loaded with a negative selective marker such as SacB gene (which encodes levansucrase) or a plasmid having a temperature-sensitive (ts) replication mechanism. Alternatively, a transformant having the expression cassette inserted at a random position on the genome may be obtained by transformation using a DNA fragment consisting of only the expression cassette.

In the case of utilizing an enzyme originally encoded on the genome (endogenous enzyme) of the host as the predetermined enzyme, its expression may be enhanced by replacing a promoter of the enzyme gene on the genome with a strong one. The promoter for use in the expression vector mentioned above can also be utilized as the promoter.

### (Cell-free protein synthesis reaction solution)

The cell-free protein synthesis system is a system in which a protein is synthesized *in vitro* using a cell-free protein synthesis reaction solution obtained by adding an amino acid, an energy molecule such as ATP, an energy regeneration system, salts such as magnesium ions, and a gene (DNA or RNA) encoding the protein to be expressed to cell-free extracts obtained by extraction from various organisms, without the use of living microbes, cells or the like. The cell-free extracts contain translational components such as ribosome, tRNA, aminoacyl tRNA synthetase, a translation initiation factor, a translation elongation factor and a translation release factor. In the present specification, a solution for performing protein synthesis reaction through the cell-free protein synthesis system is referred to as a cell-free protein synthesis reaction solution, irrespective of whether to be before or after the reaction.

The cell-free protein synthesis system used in the present invention can be any system that can express each predetermined enzyme in a state having its original functions. For example, a wheat germ-derived synthesis system, an *Escherichia* coli-derived synthesis system, a rabbit reticulocyte-derived system, an insect cell-derived synthesis system or a human cell-derived synthesis system can be used. Alternatively, a PURE (protein synthesis using recombinant elements) system or the like may be used which prepares a necessary soluble protein factor as a recombinant protein. A batch method, a CFCF (continuous-flow cell-free) method, a CECF (continuous exchange cell-free) method, an overlay method or the like can be used as a protein synthesis reaction process in the cell-free system. RNA or DNA may be used as a template for the enzyme gene to be expressed. In the case of using RNA as the template, total RNA, mRNA, an *in vitro* transcript or the like can be used.

### [Second step]

The second step is the step of preparing, if necessary, a treated product from the transformant or the cell-free protein synthesis reaction solution after the first step. Examples of the treated product of the transformant include dormant cells, cells with improved membrane permeability, inactivated cells and homogenized cells prepared from the transformant. Cell-free extracts prepared from the homogenized cells, and purified enzymes are also included in the treated product of the present invention. Examples of the treated product of the cell-free protein synthesis reaction solution include purified enzymes prepared from the cell-free protein synthesis reaction solution. In addition, stabilized products obtained by the stabilization treatment of the transformant, the cell-free protein synthesis reaction solution and their treated products are also included in the treated product of the present invention.

The dormant cells can be prepared by use of a method known in the art. The dormant cells mean microbial cells placed in a state where their proliferation has been substantially arrested. Specifically, the transformant that has proliferated by culture can be recovered from the medium and then suspended in a buffer, etc. free from a carbon source or the like easily utilizable by the transformant, or the recovered transformant can be frozen, dried and powdered to prepare dormant cells. The method for recovering the transformant from the medium can be any method. Examples thereof include a method using centrifugation and a method using membrane filtration. The centrifugation is not particularly limited as long as the centrifugation can supply centrifugal force that precipitates the transformant. For example, a tubular bowl or disc centrifuge can be utilized. The centrifugal force can be on the order of, for example, 500 G to 20,000 G. The membrane filtration may be performed using any of microfiltration (MF) and ultrafiltration (UF) membranes as long as the transformant can be recovered from the medium. The buffer for suspending the transformant can be any buffer that substantially arrests the proliferation of the transformant and maintains the functions of each predetermined enzyme. For example, a phosphate buffer, an acrylate buffer, a Tris (tris(hydroxymethyl)aminomethane)-HCl buffer, HEPES (2-(4-(2-hydroxyethyl)-1-piperazinyl)ethanesulfonic acid) or other Good's buffers can be used. In the case of freezing the transformant, the transformant can be frozen in a state where most of water has been removed by an operation such as the centrifugation described above, or in a state suspended in an appropriate buffer. The freezing temperature differs depending on components of the buffer for suspending the transformant, and can be any temperature at which the transformant is substantially frozen. The freezing can be performed in the range of, for example, -210°C to 0°C. In the case of drying the transformant, the drying method can be any method that substantially arrests the proliferation of the transformant and maintains the functions of each predetermined enzyme. Examples thereof include a freeze drying method and a spray drying method.

The cells with improved membrane permeability can be prepared by use of a method known in the art. The transformant is treated with, for example, an organic solvent or a surfactant so that a substrate or a product easily passes through the cell membrane or the cell wall of the transformant. The type of the organic solvent or the surfactant used is not particularly limited as long as the organic solvent or the surfactant improves membrane permeability and maintains the functions of each predetermined enzyme. Examples of the organic solvent include toluene and methanol. Examples of the surfactant include Triton-X 100 and benzethonium chloride.

The inactivated cells can be prepared by use of an approach known in the art. For example, chemical treatment or heat treatment can be performed. For example, a cationic surfactant such as benzethonium chloride, cetyl pyridinium chloride, methyl stearoyl chloride or cetyl trimethylammonium bromide, or a zwitterionic surfactant such as alkyldiaminoethylglycine hydrochloride can be used in the chemical treatment. Examples thereof also include alcohols such as ethanol, thiols such as 2-mercaptoethanol, amines such as ethylenediamine, and amino acids such as cysteine, ornithine and citrulline. The heat treatment can be performed using a temperature and a time that do not inactivate the enzyme of interest.

The homogenized cells can be prepared by use of an approach known in the art. Examples thereof include ultrasonication, high-pressure treatment with a French press or a homogenizer, grinding treatment with a bead mill, collision treatment with an impact crushing apparatus, enzymatic treatment using lysozyme, cellulase, pectinase or the like, freezing-thawing treatment, hypotonic solution treatment, and bacteriolysis induction treatment with phages. Any of these methods can be utilized alone or in necessary combination. In the case of homogenizing cells on an industrial scale, for example, high-pressure treatment, grinding treatment, collision treatment, or enzymatic treatment combined with any of these treatments is preferably performed in light of operability, recovery rate, cost, etc.

In the case of performing grinding treatment with a bead mill, the beads used have, for example, a density of 2.5 to 6.0 g/cm³ and a size of 0.1 to 1.0 mm, and the mill can usually be filled with approximately 80 to 85% of the beads for homogenization. Any of batch and continuous systems can be adopted to an operational system.

In the case of performing high-pressure treatment, the treatment pressure is not particularly limited as long as the protein recovery rate of interest from cells is sufficiently high. The homogenization can be performed at a pressure, for example, on the order of 40 to 200 MPa, preferably on the order of 60 to 150 MPa, more preferably on the order of 80 to 120 MPa. If necessary, multistage treatment may be performed by arranging apparatuses in series or using an apparatus having a plurality of stage structures, to improve homogenization and operation efficiency. If necessary, cooling treatment is preferably performed because the temperature usually elevates by 2 to 3°C per 10 MPa of treatment pressure.

The collision treatment can efficiently homogenize cells, for example, by preparing cell slurry in advance into frozen fine particles (e.g., 50 µm or smaller) by spray rapid freezing treatment (freezing rate: for example, several thousands of °C per minute), and colliding these particles with a collision plate using a high-speed (e.g., approximately 300 m/s) carrier gas.

The cell-free extracts can be prepared by removing homogenization residues (insoluble fractions containing cell membranes, cell walls, etc.) from the homogenized cells. The removal of the homogenization residues can be performed by an approach known in the art. For example, centrifugation, membrane filtration or filter paper filtration can be used. The centrifugation operation can be performed as mentioned above. If the homogenization residues of the transformant are fine and difficult to precipitate easily, residue precipitation efficiency may be enhanced by using an aggregating agent or the like, if necessary. The membrane filtration can also be performed as mentioned above. If the homogenization residues of the transformant are fine, particularly, an ultrafiltration (UF) membrane can be used. In the case of performing filter paper filtration, this operation can be performed by combined use with a filter aid or an aggregating agent. Examples of the filter aid include diatomaceous earth, cellulose powders and activated carbon. Examples of the aggregating agent include cationic aggregating agents, anionic aggregating agents, amphoteric aggregating agents and nonionic aggregating agents. A supernatant in which microbial cells are absent can be recovered as a cell-free supernatant by any of these operations to prepare cell-free extracts containing each predetermined enzyme.

The purified enzyme can be prepared by using general biochemical methods, for example, ammonium sulfate precipitation, various chromatography techniques (e.g., gel filtration chromatography (Sephadex column, etc.), ion exchange chromatography (DEAE-Toyopearl, etc.), affinity chromatography (TALON Metal Affinity Resin, etc.), hydrophobic chromatography (butyl Toyopearl, etc.) and anion chromatography (MonoQ column, etc.)) and SDS polyacrylamide gel electrophoresis, alone or in appropriate combination.

In the present invention, a product obtained by the stabilization treatment of the transformant, the cell-free protein synthesis reaction solution, or the treated product thereof mentioned above (stabilized product) can also be used. The stabilization treatment can be any treatment that improves the stability of each predetermined enzyme against an environmental factor (temperature, pH, chemical substance concentration, etc.), or the stability of each predetermined enzyme during preservation as compared with an untreated state. Examples thereof include embedding in a gel such as acrylamide, treatment with aldehydes such as glutaraldehyde (including CLEA: cross-linked enzyme aggregate), and treatment of supporting onto an inorganic support (alumina, silica, zeolite, diatomaceous earth, etc.).

The substrate or the product used in the present invention has relatively high polarity and might limit the rate of mass transfer in cell membranes or cell walls. Thus, in the present invention, homogenized cells, cell-free extracts, a purified enzyme, or a stabilized product thereof is particularly preferably used from the viewpoint of the permeability of the substrate or the product.

The transformant, the cell-free protein synthesis reaction solution, or the treated product thereof mentioned above can be preserved under any condition as long as the enzyme activity is retained. If desired, the solution may be frozen under appropriate conditions (e.g., -80°C to -20°C for 1 day to 1 year) and preserved until use (until the third step is carried out).

In the case of combining transformants separately containing genes encoding predetermined enzymes as mentioned above, for example, in the case of preparing and combining a transformant with enhanced expression of Nampt and Prs and a transformant with enhanced expression of Ppk, (i) these transformants may be separately treated as described above, and then, the treated products thereof can be mixed, or (ii) these transformants may be mixed and then treated together as described above.

### [Third step]

The third step is the step of bringing the transformant or the cell-free protein synthesis reaction solution after the first step, or, if necessary, the treated product thereof after the second step into contact with substrates serving as various starting materials for enzymatic reaction. In this step, the second reaction with Prs and the third reaction with Nampt are performed by coupling with the ATP regeneration reaction with Ppk. In the second reaction with Prs, the substrate is phosphorylated with ATP as a phosphate source. Also, in the third reaction with Nampt, Nampt is autophosphorylated through its own ATP hydrolytic activity. Therefore, ATP is consumed. Thus, both the reactions can be performed by coupling with the ATP regeneration reaction so that the reactions are allowed to proceed efficiently while the consumed ATP is compensated for. The Prs-mediated second reaction product phosphoribosyl pyrophosphate (PRPP) is a relatively unstable compound. Therefore, the second reaction and the third reaction can be performed in the same system so that the third reaction with Nampt is immediately performed after PRPP generation. In the present invention, ATP is not depleted because ATP is regenerated from ADP and/or AMP by the ATP regeneration reaction. However, a moderate amount of ATP needs to be added into the reaction solvent according to the ATP concentration to be maintained during the reaction. In this respect, ADP or AMP may be added instead of ATP into the reaction solvent, if necessary. This is because the added ADP or AMP is rapidly regenerated into ATP in the system by the ATP regeneration system; and this state is substantially the same as the addition of a moderate amount of ATP. Alternatively, a mixture containing these components at an arbitrary ratio may be added thereto.

The second reaction and the third reaction may be combined, if necessary, with the first reaction with Rbk coupled to the ATP regeneration reaction with Ppk. In the case of combining these reactions, the first reaction with Rbk may be first performed, and the second reaction with Prs and the third reaction with Nampt can be performed with the first reaction solution as a starting material, or the first reaction with Rbk, the second reaction with Prs and the third reaction with Nampt may be performed in the same reaction system.

The second reaction with Prs and the third reaction with Nampt are performed by bringing a transformant with enhanced expression of three enzymes Nampt, Prs and Ppk, a cell-free protein synthesis reaction solution having the three enzymes expressed, or a treated product thereof into contact with R5P, NAM, ATP and polyphosphate.

The first reaction with Rbk is performed by bringing a transformant with enhanced expression of two enzymes Rbk and Ppk, a cell-free protein synthesis reaction solution having the two enzymes expressed, or a treated product thereof into contact with ribose, ATP and polyphosphate.

The starting materials for use in the third step may be purchased from general suppliers or may be synthesized through reactions by users. For example, R5P used may be a commercially available product and is preferably synthesized by the first reaction with Rbk, i.e., by bringing a transformant with enhanced expression of two enzymes Rbk and Ppk, a cell-free protein synthesis reaction solution having the two enzymes expressed, or a treated product thereof into contact with ribose and polyphosphate, from the viewpoint of starting material cost.

The polyphosphate, one of the starting materials, is known to have various chain lengths. The chain length of the polyphosphate used in the present invention can be any chain length as long as the ATP regeneration reaction is efficiently performed. The chain length is preferably on the order of 3 to 100, more preferably on the order of 3 to 30, from the viewpoint of the viscosity of a solution of the polyphosphate dissolved therein, and cost.

In the third step, a compound other than the starting materials may be brought into contact with the transformant with enhanced expression of each predetermined enzyme, the cell-free protein synthesis reaction solution having the predetermined enzyme expressed, or the treated product thereof, or may be contained in the reaction solvent (in the production system), if necessary. For example, metal ions such as magnesium ions are appropriately contained as a component for allowing each enzyme to exert its functions. Also, a buffer component is preferably contained therein.

When the transformant used is substantially alive, i.e., when the transformant maintains the ability of cells to proliferate, the reaction is preferably performed under conditions where the transformant does not substantially proliferate. Through the reaction under such conditions, it can be expected that the product of interest is produced at high yields without utilizing the substrate or the product in this reaction in the proliferation of the transformant or degrading the substrate or the product. The conditions where the transformant does not substantially proliferate can be any condition that does not substantially increase the number of the transformant in the reaction system. Examples thereof include conditions under which the reaction is performed in a solution free from a carbon source (glucose, etc.) easily utilizable by the transformant.

The amount of the transformant with enhanced expression of each predetermined enzyme, the cell-free protein synthesis reaction solution having the predetermined enzyme expressed, or the treated product thereof, i.e., more specifically, the respective amounts of Nampt, Prs, Rbk and Ppk, contained in the reaction solvent (in the production system) can be appropriately adjusted. Likewise, the amounts of R5P, NAM, ATP, polyphosphate, ribose, and other starting materials optionally used, contained in the reaction medium can also be appropriately adjusted. The concentration of each of the substances described above in the reaction solvent (in the production system) is as follows.

The concentration of Nampt is, for example, 1 µg/L to 10 g/L. The concentration of Prs is, for example, 1 µg/L to 10 g/L. The concentration of Rbk is, for example, 1 µg/L to 10 g/L. The concentration of Ppk is, for example, 1 µg/L to 10 g/L. The concentration of each predetermined enzyme in the reaction solvent can be adjusted to the range described above by appropriately adjusting the amount of the transformant with enhanced expression of the predetermined enzyme, the cell-free protein synthesis reaction solution having the predetermined enzyme expressed, or the treated product thereof, added to the reaction solvent.

The concentration of R5P is, for example, 1 µg/L to 100 g/L. The concentration of the ribose is, for example, 1 µg/L to 100 g/L. The concentration of NAM is, for example, 1 µg/L to 500 g/L. The concentration of ATP is, for example, 1 µg/L to 100 g/L. The concentration of the polyphosphate is, for example, 1 µg/L to 200 g/L. The concentration of each starting material in the reaction solvent can be adjusted to the range described above by adjusting the amounts of these starting materials added to the reaction solvent, for example. The addition to the reaction solvent may be performed, according to the starting materials, by charging predetermined amounts of the starting materials together at the initial stage of the third step, or by adding predetermined amounts of the starting materials in order at the initial stage and/or appropriate intermediate stage of the third step.

Conditions, other than the concentration of each substance as mentioned above, for allowing enzymatic reaction to proceed, for example, a temperature and a time, can also be appropriately adjusted. The reaction temperature is preferably adjusted in a range that optimizes the catalysis efficiency of each enzyme. The reaction time can be long enough for the amount of NMN (compound of interest) generated to reach a predetermined amount.

The generated NMN can be recovered from the production system and appropriately concentrated and purified according to a routine method. The recovery and purification method used can be any method that can improve the purity of NMN and can efficiently recover NMN. Examples thereof include methods as described below. In the case of performing a reaction using microbial cells after NMN synthesis reaction, the microbial cells can be removed by an approach such as centrifugation or membrane filtration. Alternatively, in the case of performing a reaction using cell-free extracts or a purified enzyme, proteins, etc. can be removed, for example, by filtration through an ultrafiltration membrane, or by centrifugation following precipitation by the addition of perchloric acid or the like. In the case of performing a treatment with perchloric acid, pH is brought back to weakly acidic pH with potassium hydroxide or the like, and the resulting precipitates of potassium perchlorate are removed by centrifugation again. After the removal of microbial cells or proteins, washing treatment can be performed by activated carbon adsorption, if necessary. An aqueous solution containing NMN is brought into contact with activated carbon to adsorb NMN thereon. The NMN-adsorbed activated carbon can be filtered through a filter paper or the like and then washed with a solvent such as isoamyl alcohol to remove given impurities. Subsequently, the solution can be further purified by treatment with an anion exchange resin. The solution containing NMN is injected to an anion exchange resin such as Dowex, and the adsorbed NMN can be eluted with water. The pH of the obtained aqueous NMN solution is further adjusted to acidic pH, and NMN can be obtained as precipitates by the addition of acetone in a large amount. The precipitates can be dried to obtain purified NMN.

The method of the present invention can be performed such that the total sum of the respective numbers of moles of ATP, ADP and AMP to be added to the reaction system is 0.5 equivalents or less of the number of moles of NMN to be generated. Any method can be used as an approach for performing this as long as the amount of ATP, etc. added for NMN production can be consequently reduced. For example, approaches given below can be carried out alone or in appropriate combination. The details of these approaches will be described in the description about the fourth aspect mentioned later.
(1) Coupling of an ATP regeneration system
(2) Coexistence of PPase
(3) Utilization of bacterium-derived Nampt
(4) Utilization of a host having an unnecessary gene disrupted or deleted
(5) Appropriate substrate concentration

### - Second aspect -

The method for producing NMN according to the second aspect of the present invention comprises the step of bringing a transformant with enhanced expression of Nampt, a cell-free protein synthesis reaction solution having the enzyme expressed, or a treated product thereof into contact with NAM and PRPP in the presence of PPase.

The method for producing NMN according to the second aspect is carried out by performing the first step, the second step and the third step in order, as in the first aspect, except that: the first step and the second step are steps for preparing a transformant with enhanced expression of at least Nampt, a cell-free protein synthesis reaction solution having the enzyme expressed, or a treated product thereof, while preparing a transformant with enhanced expression of PPase, a microbe, etc. expressing PPase as an endogenous enzyme, albeit without enhancing its expression, a cell-free protein synthesis reaction solution having the enzyme expressed, or a treated product thereof; and in the third step, such a transformant, a cell-free protein synthesis reaction solution or a treated product thereof after the first step and the second step can be brought into contact with at least NAM and PRPP.

### PPase

PPase (EC number: 3.6.1.1) is an enzyme that hydrolyzes pyrophosphate into two molecules of phosphate. In the method for producing NMN according to the second aspect of the present invention, the third reaction can proceed very efficiently by performing the third reaction in the presence of PPase.

In the third reaction with Nampt, pyrophosphate is produced as a by-product with NMN. In view of general enzymatic reaction, the addition of PPase to the third reaction system is predicted to degrade the by-product pyrophosphate into phosphate and to accelerate the reaction for NMN generation. However, this is not necessarily obvious for Nampt. This is because Nampt reaction might not proceed continuously if pyrophosphate is degraded. Nampt is known to be activated by autophosphorylation through the hydrolysis of ATP. The dephosphorylation of autophosphorylated Nampt is necessary on a reaction basis for continuing the third reaction with Nampt. Pyrophosphate is reportedly a trigger substance for the dephosphorylation (Biochemistry 47, 11086-11096 (2008)). Thus, the possibility is quite conceivable that the removal of pyrophosphate with PPase hinders the dephosphorylation of Nampt so that the reaction is not continued. However, in the second aspect, the third reaction can be markedly accelerated by performing the third reaction in the presence of PPase.

Examples of PPase include a yeast-derived enzyme (P00817), an *Escherichia coli*-derived enzyme (NP_418647), a *Bacillus* subtilis-derived enzyme (P37487), a *Thermus thermophilus*-derived enzyme (P38576), a *Streptococcus gordonii-derived* enzyme (P95765) and *Streptococcus mutans* (O68579).

PPase can be in any form that can be added to the third reaction system, and can be prepared by any method. Specifically, as in the first step according to the first aspect, a transformant containing a gene encoding PPase is first prepared and cultured, or protein synthesis reaction is performed with a cell-free protein synthesis reaction solution containing a gene encoding the enzyme to express the enzyme. Since PPase is expressed in a given amount as an enzyme necessary for survival in usual microbes, etc., a microbe, etc. without enhanced expression of the enzyme can be cultured and directly used. Subsequently, as in the second step according to the first aspect, a treated product can be prepared from the transformant, the microbe, etc. without enhanced expression of the enzyme, or the cell-free protein synthesis reaction solution after the first step. Alternatively, a commercially available purified enzyme of PPase may be utilized as one form of the treated product. Examples of the commercially available purified enzyme of PPase include a yeast-derived purified enzyme of PPase (product No. 10108987001) from Sigma-Aldrich Co. LLC.

The method for producing NMN according to the second aspect can be carried out by bringing the transformant with enhanced expression of Nampt, the cell-free protein synthesis reaction solution having the enzyme expressed, or the treated product thereof into contact with NAM and PRPP in the presence of the PPase obtained as described above. The method of the second aspect can allow the third reaction to proceed very efficiently and can efficiently produce NMN. The third reaction according to the second aspect may be performed alone or may be performed in combination with one or more of the first reaction, the second reaction and the ATP regeneration reaction in the same reaction system. In other words, the method for producing NMN according to the first aspect and the second aspect integrated with each other may be carried out by designing the third reaction defined in the second aspect of the present invention as an embodiment of the third reaction according to the first aspect of the present invention.

When the transformant used is substantially alive, i.e., when the transformant maintains the ability of cells to proliferate, the reaction is preferably performed under conditions where the transformant does not substantially proliferate. Through the reaction under such conditions, it can be expected that the product of interest is produced at high yields without utilizing the substrate or the product in this reaction in the proliferation of the transformant or degrading the substrate or the product. The conditions where the transformant does not substantially proliferate can be any condition that does not substantially increase the number of the transformant in the reaction system. Examples thereof include conditions under which the reaction is performed in a solution free from a carbon source (glucose, etc.) easily utilizable by the transformant.

In a mode of the second step according to the present invention, the treated product is particularly preferably a purified enzyme. Use of the purified enzyme can suppress the degradation or side reaction of the reactant (substrate) or the product. Therefore, the effect of accelerating the third reaction according to the present invention can be more enjoyed.

### - Third aspect -

The method for producing NMN according to the third aspect of the present invention comprises the step of bringing a transformant with enhanced expression of nicotinamide phosphoribosyltransferase (Nampt) or a treated product thereof into contact with at least nicotinamide (NAM), wherein the transformant has disruption or deletion of a gene encoding an enzyme classified into EC number represented by (d) EC 3.5.1.42, and a gene encoding an enzyme classified into at least any one of EC numbers represented by the following (a), (c), (g), (h) and (i) :
(a) EC 3.1.3.5,
(c) EC 2.4.2.1,
(g) EC 3.2.2.1,
(h) EC 3.2.2.3, and
(i) EC 3.2.2.14.

The method for producing NMN according to the third aspect is carried out by performing the first step, the second step and the third step in order, as in the first aspect, except that: the first step and the second step are steps for preparing a transformant with enhanced expression of nicotinamide phosphoribosyltransferase (Nampt) or a treated product thereof, wherein the transformant has disruption or deletion of a gene encoding an enzyme classified into each EC number; and in the third step, such a transformant or a treated product thereof after the first step and the second step can be brought into contact with at least NAM.

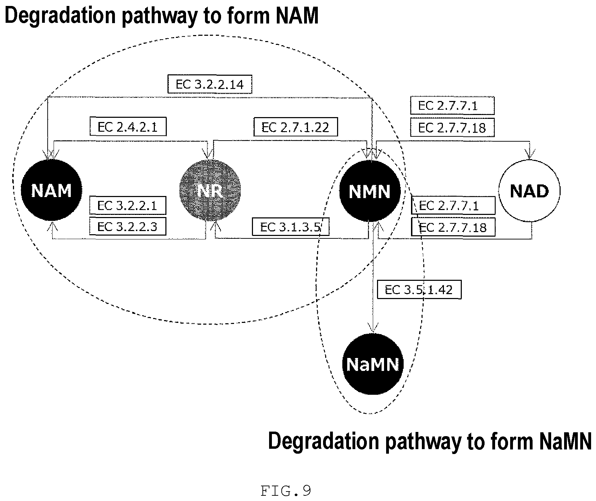

The enzyme classified into each EC number is as mentioned above. In the case of synthesizing NMN using a transformant such as *Escherichia coli* or a treated product thereof, these enzymes degrade generated NMN and are consequently responsible for reduction in the amount of NMN produced. Two NMN degradation pathways carried by hosts are known, i.e., a degradation pathway to form NAM and a degradation pathway to form nicotinic acid mononucleotide (NaMN) (Figure 9). The present inventors have completed the third aspect by finding that the degradation of NMN can be drastically suppressed by attenuating both the pathways (disrupting or deleting genes encoding one or more enzymes on the pathways) rather than by attenuating one of the pathways.

In order to attenuate the degradation pathway to form NaMN from NMN, the gene encoding an enzyme classified into EC number represented by (d) EC 3.5.1.42 can be disrupted or deleted, as shown in Figure 9.

In order to attenuate the degradation pathway to form NAM from NMN, the gene encoding an enzyme classified into at least any one of EC numbers represented by the following (a), (c), (g), (h) and (i) can be disrupted or deleted, as shown in Figure 9:
(a) EC 3.1.3.5,
(c) EC 2.4.2.1,
(g) EC 3.2.2.1,
(h) EC 3.2.2.3, and
(i) EC 3.2.2.14.

The gene encoding an enzyme classified into EC number represented by (a) EC 3.1.3.5 or (i) EC 3.2.2.14 is preferably disrupted or deleted because the enzyme is directly involved in the degradation of NMN. More preferably, the gene encoding an enzyme classified into EC number represented by (a) EC 3.1.3.5 is disrupted or deleted.

The method for disrupting or deleting each gene is not particularly limited and is as described in the description about the first aspect. The method for producing NMN may be carried out by integrating the third aspect of the present invention with one or both of the first and second aspects of the present invention.

### - Fourth aspect -

In the method for producing NMN according to the fourth aspect of the present invention, the total sum of the respective numbers of moles of ATP, ADP and AMP to be added to the reaction system for NMN production can be 0.5 equivalents or less of the number of moles of NMN to be generated.

ATP is required for the production of NMN through the first reaction, the second reaction and the third reaction mentioned above. Specifically, in the first reaction, 1 mol of ATP is used per mol of R5P to be generated because ATP is converted to ADP along with the generation of R5P from ribose. In the second reaction, 1 mol of ATP is used per mol of PRPP to be generated because ATP is converted to AMP along with the generation of PRPP from R5P. In the third reaction, ATP is not essential for the reaction itself to generate NMN from PRPP. However, as mentioned above, Nampt has ATP hydrolytic activity, and enzymatic parameters or chemical equilibrium shifts in favor of NMN generation by the autophosphorylation of Nampt through the hydrolysis of ATP. Thus, when the third reaction system is rich in ATP, 1 mol or more of ATP is substantially used per mol of PRPP to be generated along with the generation of NMN from PRPP. Specifically, in the production of NMN by the second reaction and the third reaction with R5P as a starting material, 2 mol or more in total of ATP is used per mol of NMN to be generated. In the production of NMN by the first reaction, the second reaction and the third reaction with ribose as a starting material, 3 mol or more in total of ATP is used per mol of NMN to be generated.

Since ATP is an expensive compound, it is desirable to minimize the amount of ATP used for inexpensively producing NMN. Through the use of the method for producing NMN according to the fourth aspect of the present invention, the total sum of the respective numbers of moles of ATP, ADP and AMP to be added to the reaction system for NMN production can be 0.5 equivalents or less of the number of moles of NMN to be generated.

The method for producing NMN according to the fourth aspect can be performed by use of any method as long as the amount of ATP, etc. added for NMN production can be reduced. For example, approaches given below can be carried out alone or in appropriate combination.

### (1) Coupling of ATP regeneration system

A system that can regenerate the by-product ADP or AMP into ATP can be coupled to at least a NMN generation reaction system involving the second reaction and the third reaction, to reduce the total sum of the respective numbers of moles of ATP, ADP and AMP to be added to the reaction system for NMN production. The ATP regeneration system can be any system that can regenerate ATP from AMP and ADP. Examples thereof include a system using Ppk with polyphosphate as a phosphate source, a system using pyruvate kinase with phosphoenolpyruvic acid as a phosphate source, and a system using creatine phosphate kinase with creatine phosphate as a phosphate source. A system using Ppk with polyphosphate as a phosphate source is preferred from the viewpoint of phosphate source cost. Specifically, the NMN generation reaction coupled to the ATP regeneration system using polyphosphate and Ppk can be carried out as described in the third step according to the first aspect.

### (2) Coexistence of PPase

The coexistence of PPase with the NMN generation reaction system degrades the by-product pyrophosphate into phosphate and accelerates the reaction for NMN generation. As a result, the total sum of the respective numbers of moles of ATP, ADP and AMP to be added to the reaction system for NMN production can be reduced. Specifically, the NMN generation reaction with which PPase coexists can be carried out as described in the second aspect.

### (3) Utilization of bacterium-derived Nampt

Use of bacterium-derived Nampt as the enzyme Nampt that catalyzes the third reaction allows NMN generation to proceed efficiently. As a result, the total sum of the respective numbers of moles of ATP, ADP and AMP to be added to the reaction system for NMN production can be reduced. The bacterium-derived Nampt described in the first step according to the first aspect can be utilized.

### (4) Utilization of host having unnecessary gene disrupted or deleted

In the case of performing NMN generation reaction using a transformant, dormant cells, cells with improved membrane permeability, inactivated cells or homogenized cells prepared from the transformant, cell-free extracts prepared from the homogenized cells, or a stabilized product obtained by stabilization treatment thereof, a host can be used which has disruption or deletion of a gene responsible for the degradation or side reaction of the reactant (substrate) or the product. Specifically, a host can be used which has disruption or deletion of any one or more of the genes described in the first step according to the first aspect. Preferably, a host can be used which has disruption or deletion of the genes described in the third aspect.

### (5) Appropriate substrate concentration

Improvement in NMN generation rate or the amount of NMN generated can be expected by increasing a ribose or NAM concentration in the reaction system, from the viewpoint of chemical equilibrium or the substrate affinity of an enzyme. As a result, the total sum of the respective numbers of moles of ATP, ADP and AMP to be added to the reaction system for NMN production can be reduced. On the other hand, similar effects can be expected by increasing an ATP concentration in the reaction system. However, the addition of ATP in an amount more than necessary increases the number of moles of ATP used for generating 1 mol of NMN unless the amount of NMN generated is increased to an amount commensurate therewith. Specifically, NMN can be efficiently produced by the addition of ATP in an appropriate amount to the reaction system. The number of moles of ATP to be added to the reaction system is preferably 1 equivalent or less, more preferably 0.5 equivalents or less, further preferably 0.1 equivalents or less, of the number of moles of NMN to be generated.

The method for producing NMN may be carried out by integrating the fourth aspect of the present invention with one or two or more of the first, second and third aspects of the present invention.

### Examples

### [Example 1] <NMN synthesis from ribose through use of ATP regeneration system>

In this Example, the predetermined enzymes used were the following enzymes.
Nampt: *Haemophilus ducreyi-derived* (AAR87771)
Prs: *Bacillus* subtilis-derived (BAA05286) and *Homo* sapiens-derived (NP_002755)
Ppk (Ppk2 class 3): *Deinococcus radiodurans-*derived (NP_293858)
Rbk: *Saccharomyces cerevisiae-derived* (P25332)

### (1) Preparation of expression plasmid

An expression plasmid for each enzyme was prepared as described below. For each enzyme derived from an organism species described in "Origin" of Table 1, DNA (which consisted of the nucleotide sequence represented by SEQ ID NO described in "Nucleotide sequence" of Table 1) encoding the enzyme protein consisting of the amino acid sequence represented by SEQ ID NO described in "Amino acid sequence" in the table was synthesized and cloned into the NdeI-XhoI site of expression vector pET-26b(+) (Novagen) (the gene synthesis was carried out by GenScript Japan Inc., and codons were optimized for expression in *Escherichia coli*). Each plasmid thus obtained was designated as shown in "Expression plasmid" of Table 1.

**Table 1**

| Enzyme | Origin | Amino acid sequence | Nucleotide sequence | Expression plasmid |
|---|---|---|---|---|
| Nampt | Haemophilus ducreyi | SEQ ID NO: 1 | SEQ ID NO: 2 | pEHdNampt |
| Prs | Bacillus subtilis | SEQ ID NO: 3 | SEQ ID NO: 4 | pEBsPrs |
| | Homo sapiens | SEQ ID NO: 5 | SEQ ID NO: 6 | pEHsPrs |
| Rbk | Saccharomyces cerevisiae | SEQ ID NO: 7 | SEQ ID NO: 8 | pEScRbk |
| Ppk | Deinococcus radiodurans | SEQ ID NO: 9 | SEQ ID NO: 10 | pEDrPpk |

### (2) Preparation of transformant with enhanced expression of each enzyme

Competent cells (Zip Competent Cell BL21 (DE3), Funakoshi Co., Ltd.) of an *Escherichia coli* BL21 (DE3) strain were thawed on ice, mixed with each plasmid DNA solution prepared in (1), and left standing on ice for 10 minutes. After application of heat shock at 42°C for 45 seconds, the cells were ice-cooled again, and SOC medium was added thereto. The cells were shake-cultured at 37°C for 1 hour, then spread over LB agar medium (containing 50 mg/L kanamycin sulfate), and statically cultured overnight at 37°C. The obtained colony was used as a recombinant with enhanced expression of each enzyme.

### (3) Culture of recombinant

The colony of the recombinant with enhanced expression of each enzyme was inoculated to 2 ml of LB medium (containing 50 mg/L kanamycin sulfate) supplemented with Overnight Express Autoinduction system 1 (Merck KGaA) according to the attached protocol. The colony was shake-cultured at 200 rpm at a temperature of 37°C for 3 hours and then further shake-cultured for 18 hours with the conditions changed to 200 rpm at a temperature of 17°C.

### (4) Preparation of cell-free extracts

The culture solution obtained in (3) was centrifuged (5,000 × g, 10 min), and the supernatant was discarded. A 50 mM HEPES-NaOH buffer (pH 7.5) was added to precipitated microbial cells to adjust turbidity at a wavelength of 630 nm to 10. However, this procedure was performed using a 50 mM potassium phosphate buffer (pH 7.5) as to the Prs-expressing microbial cells. 0.5 ml of the suspension of the microbial cells in the buffer was homogenized with Bioruptor (Cosmo Bio Co., Ltd.) for 15 minutes. The homogenate was centrifuged (5,000 × g, 10 min), and the obtained supernatant was used as cell-free extracts. The protein concentration of the cell-free extracts was measured by use of Bio-Rad protein assay (Bio-Rad Laboratories, Inc.) with BSA (bovine serum albumin, Bio-Rad Laboratories, Inc.) as a standard protein.

### (5) NMN synthesis reaction

NMN synthesis reaction was performed using the cell-free extracts prepared in (4). The amount of the reaction solution was set to 100 µL. Each reaction solution was prepared according to the composition shown in Table 2 (Nos. 1-2 and 1-4) and statically reacted at 37°C.

**Table 2**

| Reaction solution composition (final concentration) | | Reaction sample | | | |
|---|---|---|---|---|---|
| | | No. 1-1 | No. 1-2 | No. 1-3 | No. 1-4 |
| | | Prs: BsPrs | | Prs: HsPrs | |
| | | without Ppk | with Ppk | without Ppk | with Ppk |
| HEPES-NaOH (pH7.5) [mM] | | 50 | 50 | 50 | 50 |
| Nicotinamide [mM] | | 2 | 2 | 2 | 2 |
| Ribose [mM] | | 10 | 10 | 10 | 10 |
| Magnesium chloride [mM] | | 10 | 10 | 10 | 10 |
| Polyphosphate [mM] | | 1 | 1 | 1 | 1 |
| Adenosine triphosphate [mM] | | 0.1 | 0.1 | 0.1 | 0.1 |
| Potassium phosphate buffer [mM] | | 5 | 5 | 5 | 5 |
| Dithiothreitol [mM] | | 1 | 1 | 1 | 1 |
| Ammonium sulfate [mM] | | 20 | 20 | 20 | 20 |
| Cell-free extrac ts | ScRbk [g/L] | 0.03 | 0.03 | 0.03 | 0.03 |
| | BsPrs [g/L] | 0.4 | 0.4 | - | - |
| | HsPrs [g/L] | - | - | 0.5 | 0.5 |
| | HdNampt [g/L] | 0.3 | 0.3 | 0.3 | 0.3 |
| | DrPpk [g/L] | - | 1.4 | - | 1.4 |

10 µL of the reaction solution was sampled immediately after the start of the reaction (0 hours), 3 hours later and 6 hours later. The sampled reaction solution was mixed with 90 µL of an HPLC mobile phase described in (6), and the mixture was rapidly ice-cooled to terminate the reaction. The diluted solution was filtered (5,000 × g, 10 min) through an ultrafiltration membrane (Centricut Ultramini, molecular weight cutoff: 10,000, Kurabo Industries Ltd.), and the filtrate was analyzed by HPLC.

### (6) Analysis of NMN

The NMN synthesis reaction sample was analyzed by HPLC under the following conditions.
Column: SUPELCOSIL LC-18-T (Sigma-Aldrich Co. LLC) Mobile phase: 0.05 M KH₂PO₄/K₂HPO₄ (pH 7)
Flow rate: 1 ml/min
Detection: UV 261 nm
Column temperature: 30°C

### (7) Experimental results

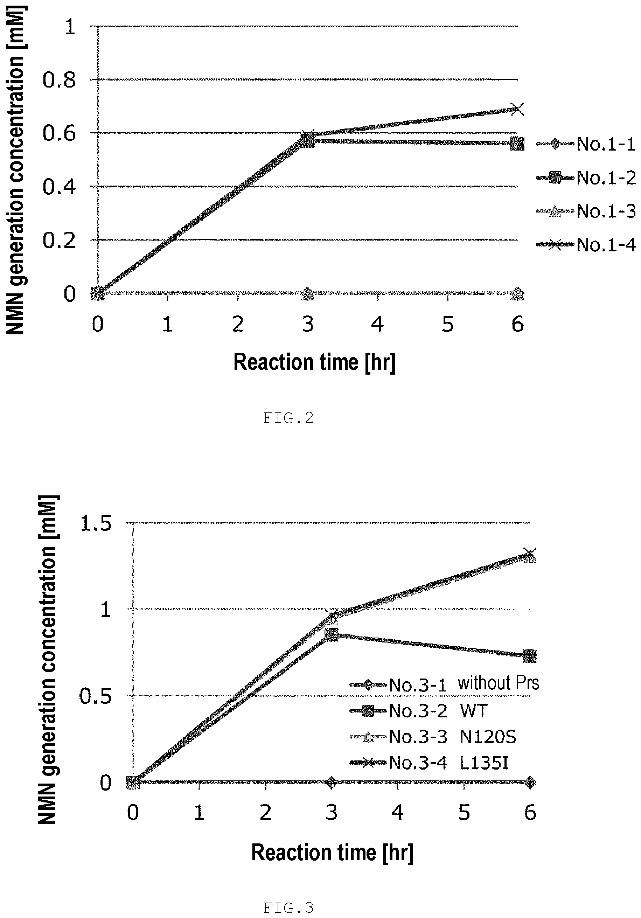

The experimental results are shown in Figure 2. The generation of 0.5 mM and 0.6 mM NMN was observed in sample No. 1-2 and No. 1-4, respectively, supplemented with Ppk. The number of moles of the added ATP (0.01 µmol) was 0.2 equivalents and 0.17 equivalents, respectively, of the number of moles of the generated NMN (0.05 µmol and 0.06 µmol). *Bacillus subtilis*-derived Prs (BsPrs) and *Homo* sapiens-derived Prs (HsPrs) were both confirmed to generate NMN. These results demonstrated that NMN can be efficiently synthesized from ribose by the coexistence of the ATP-regenerating enzyme Ppk.

### [Comparative Example 1] <NMN synthesis from ribose without use of ATP regeneration system>

### (1) Preparation of recombinant, culture and preparation of cell-free extracts

In this Comparative Example, cell-free extracts of each enzyme were prepared by the same operation as in Examples 1(2) to 1(4).

### (2) NMN synthesis reaction and analysis of NMN

NMN synthesis reaction and analysis were performed using the cell-free extracts obtained in (1). The amount of the reaction solution was set to 100 µL, and the procedures were performed in the same way as in Examples 1(5) and 1(6) except that each reaction solution was prepared according to the composition shown in Table 2 (Nos. 1-1 and 1-3).

### (3) Experimental results

The experimental results are shown in Figure 2. NMN was generated at a level equal to or lower than the detection limit in sample No. 1-1 and No. 1-3 supplemented with no Ppk.

### [Example 2] <Synthesis of NMN with cryopreserved cell-free extracts>

### (1) Preparation of recombinant, culture and preparation of cell-free extracts

In this Example, cell-free extracts of each enzyme were prepared by the same operation as in Examples 1(2) to 1(4) except that only *Bacillus subtilis*-derived Prs (BsPrs) was used as Prs. NMN synthesis reaction mentioned later in (2) was performed immediately after the preparation. The obtained cell-free extracts of each enzyme were preserved at -20°C. One month later, NMN synthesis reaction was performed again using the preserved cell-free extracts.

### (2) NMN synthesis reaction and analysis of NMN

NMN synthesis reaction and analysis were performed using the cell-free extracts immediately after the preparation and after the 1-month preservation (preservation at -20°C) obtained in (1). The procedures were performed in the same way as in Examples 1(5) and 1(6) except that: each reaction solution was prepared according to the composition shown in Table 3; and the reaction solution was sampled 6 hours after the start of the reaction.

**Table 3**

| Reaction solution composition (final concentration) | | Reaction sample | |
|---|---|---|---|
| | | No. 2-1 | No. 2-2 |
| | | Immediately after preparation of cell-free extracts | After preservation at -20°C of cell-free extracts |
| HEPES-NaOH (pH7.5) [mM] | | 50 | 50 |
| Nicotinamide [mM] | | 2 | 2 |
| Ribose [mM] | | 10 | 10 |
| Magnesium chloride [mM] | | 10 | 10 |
| Polyphosphate [mM] | | 1 | 1 |
| Adenosine triphosphate [mM] | | 0.1 | 0.1 |
| Potassium phosphate buffer [mM] | | 5 | 5 |
| Dithiothreitol [mM] | | 1 | 1 |
| Ammonium sulfate [mM] | | 20 | 20 |
| Cell-free extract s | ScRbk [g/L] | 0.03 | 0.03 |
| | BsPrs [g/L] | 0.4 | 0.4 |
| | HdNampt [g/L] | 0.3 | 0.3 |
| | DrPpk [g/L] | 1.4 | 1.4 |

### (3) Experimental results

The generation of 0.5 mM NMN was observed 6 hours after the start of the reaction in sample No. 2-2 of the cell-free extracts preserved at -20°C, as in sample No. 2-1 immediately after the preparation of the cell-free extracts. The number of moles of the added ATP (0.01 µmol) was 0.2 equivalents of the number of moles of the generated NMN (0.05 µmol). These results demonstrated that cryopreservation allows the cell-free extracts to be preserved in a state having the ability to synthesize NMN.

### [Example 3] <Synthesis of NMN through use of Prs mutant>

In this Example, the predetermined enzymes used were the following enzymes.
Nampt: *Haemophilus ducreyi-derived* (AAR87771)
Prs: *Bacillus subtilis*-derived (BAA05286) Asn120Ser mutant (BsPrsN120S) and Leu135Ile mutant (BsPrsL135I)
Ppk (Ppk2 class 3): *Deinococcus radiodurans-*derived (NP_293858)
Rbk: *Saccharomyces cerevisiae-derived* (P25332)
BsPrsN120S and BsPrsL135I had serine replaced for asparagine at position 120 and isoleucine replaced for leucine at position 135, respectively. The organism species from which the mutants were derived, SEQ ID NOs representing the amino acid sequences of the mutants, SEQ ID NOs representing the nucleotide sequences of their DNAs, and expression plasmid names are each shown in Table 4.

**Table 4**

| Enzyme | Origin | Amino acid sequence | Nucleotide sequence | Expression plasmid |
|---|---|---|---|---|
| Prs | Bacillus subtilis | SEQ ID NO: 11 | SEQ ID NO: 12 | pEBsPrsN120S |
| | | SEQ ID NO: 13 | SEQ ID NO: 14 | pEBsPrsL135I |

### (1) Preparation of Prs mutant expression plasmid

An expression plasmid for each Prs mutant was prepared as described below. Mutagenesis PCR reaction was performed with pEBsPrs shown in Table 1 as template. Mutagenesis primer names, SEQ ID NOs representing the nucleotide sequences of the primers, and Prs mutant names are shown in Table 5.

**Table 5**

| Primer for mutagenesis | | | Prs mutant name |
|---|---|---|---|
| Forward | Prs_Bs_N120S-F | SEQ ID NO: 15 | BsPrsN120S |
| Reverse | Prs_Bs_N120S-R | SEQ ID NO: 16 | |
| Forward | Prs_Bs_L135I-F | SEQ ID NO: 17 | BsPrsL135I |
| Reverse | Prs_Bs_L135I-R | SEQ ID NO: 18 | |

The mutagenesis PCR reaction was performed according to the reaction solution composition shown in Table 6 and the reaction conditions shown in Table 7.

**Table 6**

| Component | Final concentration |
|---|---|
| Template plasmid | 20 pg/µL |
| Forward primer | 0.2 µM |
| Reverese primer | 0.2 µM |
| PrimeSTAR Max Premix (2X) (Takara Bio Inc.) | 1X |

**Table 7**

| Temperature | Time | The number of cycles |
|---|---|---|
| 98°C | 10 sec | 30 |
| 55°C | 5 sec | |
| 72°C | 30 sec | |

After the completion of the PCR reaction, each PCR product was purified using QIAquick PCR Purification Kit (Qiagen N.V.) according to the attached protocol. The generated PCR product was digested with DpnI (New England Biolabs Inc.), and *Escherichia coli* HST08 (Takara Bio Inc.) was transformed with the resulting reaction solution. A plasmid was extracted from a colony that appeared, and sequenced using primers T7-PP (SEQ ID NO: 19) and T7-TP (SEQ ID NO: 20). Each plasmid with the mutation correctly introduced was used as each mutant Prs expression plasmid shown in Table 4.

### (2) Preparation of recombinant, culture and preparation of cell-free extracts

Cell-free extracts of each enzyme were prepared by the same operation as in Examples 1(2) to 1(4) except that BsPrsN120S and BsPrsL135I were used as Prs.

### (3) NMN synthesis reaction and analysis of NMN

NMN synthesis reaction and analysis were performed using the cell-free extracts obtained in (1). The procedures were performed in the same way as in Examples 1(5) and 1(6) except that each reaction solution was prepared according to the composition shown in Table 8.

**Table 8**

| Reaction solution composition (final concentration) | | Reaction sample | | | |
|---|---|---|---|---|---|
| | | No. 3-1 | No. 3-2 | No. 3-3 | No. 3-4 |
| | | ATP 1 mM | | | |
| | | without Prs | BsPrs | | |
| | | | Wild type | N120S | L135I |
| HEPES-NaOH (pH7.5) [mM] | | 50 | 50 | 50 | 50 |
| Nicotinamide [mM] | | 2 | 2 | 2 | 2 |
| Ribose [mM] | | 10 | 10 | 10 | 10 |
| Magnesium chloride [mM] | | 10 | 10 | 10 | 10 |
| Polyphosphate [mM] | | 1 | 1 | 1 | 1 |
| Adenosine triphosphate [mM] | | 1 | 1 | 1 | 1 |
| Potassium phosphate buffer [mM] | | 5 | 5 | 5 | 5 |
| Dithiothreitol [mM] | | 1 | 1 | 1 | 1 |
| Ammonium sulfate [mM] | | 20 | 20 | 20 | 20 |
| Cell-free extrac ts | ScRbk [g/L] | 0.03 | 0.03 | 0.03 | 0.03 |
| | BsPrs [g/L] | 0.2 | - | - | - |
| | BsPrsN120S [g/L] | - | 0.2 | - | - |
| | BsPrsL135I [g/L] | - | - | 0.2 | - |
| | HdNampt [g/L] | 0.3 | 0.3 | 0.3 | 0.3 |
| | DrPpk [g/L] | 1.4 | 1.4 | 1.4 | 1.4 |

### (4) Experimental results

The experimental results are shown in Figure 3. In the case of using BsPrsN120S and BsPrsL135I, a higher amount of NMN generated than that of the wild type was exhibited 6 hours after the start of the reaction. The generation of NMN was not observed when Prs was not added. These results demonstrated that NMN can be efficiently synthesized by using a Prs mutant.

### [Example 4] <Study on substrate concentration>

### (1) Preparation of recombinant, culture and preparation of cell-free extracts

In this Example, cell-free extracts of each enzyme were prepared by the same operation as in Examples 1(2) to 1(4) except that BsPrsN120S described in Example 3 was used as Prs.

### (2) NMN synthesis reaction and analysis of NMN

NMN synthesis reaction and analysis were performed using the cell-free extracts obtained in (1). The procedures were performed in the same way as in Examples 1(5) and 1(6) except that: each reaction solution was prepared according to the composition shown in Table 9; and the reaction solution was sampled 18 hours after the start of the reaction.

**Table 9**

| Reaction solution composition (final concentration) | | Reaction sample | | | | | | | | | | | |
|---|---|---|---|---|---|---|---|---|---|---|---|---|---|
| | | No. 4-1 | No. 4-2 | No. 4-3 | No. 4-4 | No. 4-5 | No. 4-6 | No. 4-7 | No. 4-8 | No. 4-9 | No. 4-10 | No. 4-11 | No. 4-12 |
| | | Poly-P 5 mM | | | | | | Poly-P 10 mM | | | | | |
| | | MgCl₂ 10 mM | | | MgCl₂ 20 mM | | | MgCl₂ 10 mM | | | MgCl₂ 20 mM | | |
| | | R30* N6* | R30* N20* | R60* N40* | R30* N6* | R30* N20* | R60* N40* | R30* N6* | R30* N20* | R60* N40* | R30* N6* | R30* N20* | R60* N40* |
| HEPES-NaOH(pH7.5) [mM] | | 50 | 50 | 50 | 50 | 50 | 50 | 50 | 50 | 50 | 50 | 50 | 50 |
| Nicotinamide [mM] | | 6 | 20 | 40 | 6 | 20 | 40 | 6 | 20 | 40 | 6 | 20 | 40 |
| Ribose [mM] | | 30 | 30 | 60 | 30 | 30 | 60 | 30 | 30 | 60 | 30 | 30 | 60 |
| Magnesium chloride [mM] | | 10 | 10 | 10 | 20 | 20 | 20 | 10 | 10 | 10 | 20 | 20 | 20 |
| Polyphosphate [mM] | | 5 | 5 | 5 | 5 | 5 | 5 | 10 | 10 | 10 | 10 | 10 | 10 |
| Adenosine triphosphate [mM] | | 1 | 1 | 1 | 1 | 1 | 1 | 1 | 1 | 1 | 1 | 1 | 1 |
| Potassium phosphate buffer [mM] | | 5 | 5 | 5 | 5 | 5 | 5 | 5 | 5 | 5 | 5 | 5 | 5 |
| Dithiothreitol [mM] | | 1 | 1 | 1 | 1 | 1 | 1 | 1 | 1 | 1 | 1 | 1 | 1 |
| Ammonium sulfate [mM] | | 20 | 20 | 20 | 20 | 20 | 20 | 20 | 20 | 20 | 20 | 20 | 20 |
| Cell-free extra cts | ScRbk [g/L] | 0.03 | 0.03 | 0.03 | 0.03 | 0.03 | 0.03 | 0.03 | 0.03 | 0.03 | 0.03 | 0.03 | 0.03 |
| | BsPrsN12 0S [g/L] | 0.2 | 0.2 | 0.2 | 0.2 | 0.2 | 0.2 | 0.2 | 0.2 | 0.2 | 0.2 | 0.2 | 0.2 |
| | HdNampt [g/L] | 0.3 | 0.3 | 0.3 | 0.3 | 0.3 | 0.3 | 0.3 | 0.3 | 0.3 | 0.3 | 0.3 | 0.3 |
| | DrPpk [g/L] | 1.4 | 1.4 | 1.4 | 1.4 | 1.4 | 1.4 | 1.4 | 1.4 | 1.4 | 1.4 | 1.4 | 1.4 |

| | | | | | | | | | | | | | |
|---|---|---|---|---|---|---|---|---|---|---|---|---|---|
| *R represents ribose, N represents nicotinamide, and number represents final concentration (mM). | | | | | | | | | | | | | |

### (3) Experimental results

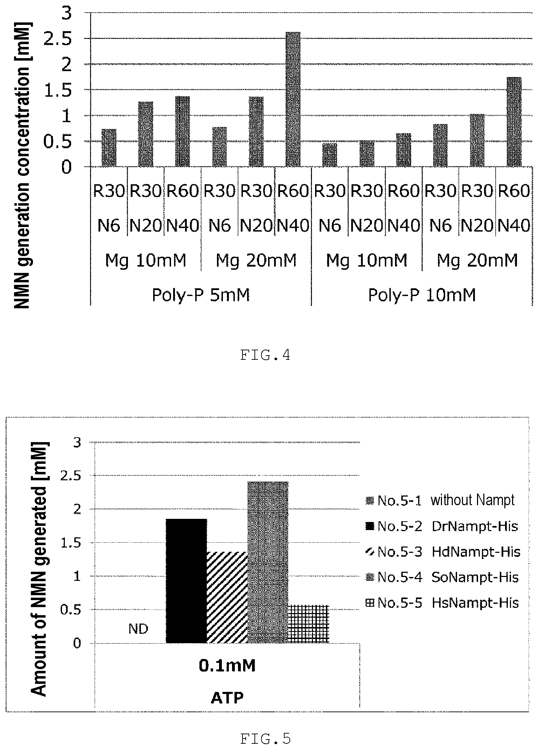

The experimental results are shown in Figure 4. The amount of NMN generated was confirmed to be large as a whole when the polyphosphate (Poly-P) concentration was 5 mM rather than 10 mM. At the polyphosphate concentration of 5 mM and the substrate ribose concentration of 30 mM, the amount of NMN generated was confirmed to be at least 1.7 times higher when the concentration of another substrate nicotinamide was 20 mM (sample Nos. 4-2 and 4-5) rather than 6 mM (sample Nos. 4-1 and 4-4). However, the influence of the magnesium concentration was hardly seen. On the other hand, when both the substrates have a 2-fold concentration at the same ratio (sample Nos. 4-3 and 4-6), the amount of NMN generated was increased merely slightly at the magnesium concentration of 10 mM (sample No. 4-3), whereas the amount of NMN generated was increased to 2.6 mM at the magnesium concentration of 20 mM (sample No. 4-6). For sample No. 4-6, the number of moles of the added ATP (0.1 µmol) was 0.38 equivalents of the number of moles of the generated NMN (0.26 µmol). These results demonstrated that for efficient generation of NMN, it is important to appropriately set the concentrations of ribose, nicotinamide, polyphosphate and magnesium.

### [Example 5] <NMN synthesis using purified enzymes of bacterium-derived and human-derived Nampt>

In this Example, the predetermined enzymes used were the following enzymes.
Nampt: His-tagged *Haemophilus ducreyi-derived* (AAR87771) Nampt (HdNampt-His), His-tagged *Deinococcus radiodurans-derived* (AE001890) Nampt (DrNampt-His), His-tagged *Shewanella oneidensis-derived* (NP_717588) Nampt (SoNampt-His) and His-tagged human *(Homo sapiens)-derived* (NP_005737) Nampt (HsNampt-His)
Prs: Asn120Ser mutant of *Bacillus subtilis-derived* (BAA05286) Prs (BsPrsN120S)
Ppk (Ppk2 class 3): *Deinococcus radiodurans-*derived (NP_293858) Ppk
Rbk: *Saccharomyces cerevisiae-derived* (P25332) Rbk

### (1) Preparation of His-tagged Nampt expression plasmid

An expression plasmid for a His tagged protein of each bacterium-derived Nampt was prepared as described below. For each of *Deinococcus radiodurans-derived* (AE001890) and *Shewanella oneidensis-derived* (NP_717588) enzymes, DNA (which consisted of the nucleotide sequence represented by SEQ ID NO described in "Nucleotide sequence" of Table 10) encoding the enzyme protein consisting of the amino acid sequence represented by SEQ ID NO described in "Amino acid sequence" in Table 10 was first synthesized and cloned into the NdeI-XhoI site of expression vector pET-26b(+) (Novagen) (the gene synthesis was carried out by GenScript Japan Inc.). Each plasmid thus obtained was designated as shown in "Expression plasmid" of Table 10.

**Table 10**

| Enzyme | Origin | Amino acid sequence | Nucleotide sequence | Expression plasmid |
|---|---|---|---|---|
| Nampt | Deinococcus radiodurans | SEQ ID NO: 21 | SEQ ID NO: 22 | pEDrNampt |
| | Shewanella oneidensis | SEQ ID NO: 23 | SEQ ID NO: 24 | pESoNampt |

Subsequently, mutagenesis PCR reaction for Histagging Nampt was performed with the prepared pEDrNampt, pESoNampt and pEHdNampt (described in Example 1) as templates. Template plasmid names, primer names for mutagenesis, SEQ ID NOs representing the nucleotide sequences of the primers, and His-tagged Nampt expression plasmid names are shown in Table 11.

**Table 11**

| Template plasmid | Primer for mutagenesis | | His-tagged Nampt expression plasmid |
|---|---|---|---|
| pEHdNampt | HdNampt-His F | SEQ ID NO: 25 | pEHdNampt-His |
| | HdNampt-His R | SEQ ID NO: 26 | |
| pEDrNampt | DrNampt-His F | SEQ ID NO: 27 | pEDrNampt-His |
| | DrNampt-His R | SEQ ID NO: 28 | |
| pESoNampt | SoNampt-His F | SEQ ID NO: 29 | pESoNampt-His |
| | SoNampt-His R | SEQ ID NO: 30 | |

The preparation of each His-tagged Nampt expression plasmid from mutagenesis PCR reaction to sequencing was performed by the same operation as in Example 3(1) except that various template plasmids and mutagenesis primers shown in Table 11 were used. A correctly His-tagged plasmid was used as each plasmid shown in Table 11.

### (2) Preparation of recombinant, culture and preparation of cell-free extracts

Preparation of a recombinant expressing each enzyme, culture and preparation of cell-free extracts were performed by the same operation as in Example 4(1). However, the recombinant as to bacterium-derived Nampt was prepared and cultured using the His-tagged expression plasmid prepared in (1) to prepare cell-free extracts containing His-tagged Nampt. In this respect, a 50 mM HEPES-NaOH buffer (pH 8.0) was used as a buffer for suspending microbial cells.

### (3) Preparation of purified enzyme of Nampt

His-tagged Nampt was purified by immobilized metal affinity chromatography using the cell-free extracts containing His-tagged bacterium-derived Nampt, prepared in (2). 200 µL of TALON Metal Affinity Resin (Takara Bio Inc.) was collected into a 1.5 ml tube, and the resin was precipitated by centrifugation (5000 g, 2 min). The supernatant was discarded, and the precipitates were suspended by the addition of 1 ml of distilled water, followed by centrifugation again. This series of washing operations were performed twice in total. Then, the resin was washed twice in the same was as above using a 50 mM HEPES-NaOH buffer (pH 8.0). 1 ml of the cell-free extracts of each His-tagged bacterium-derived Nampt was added to the resin thus washed, and gently shaken at 4°C for 1 hour. After removal of the cell-free extracts by centrifugation, the residue was washed twice using a wash buffer (50 mM sodium phosphate, 300 mM sodium chloride, pH 7.0). After removal of the wash buffer by centrifugation, 100 µL of an elution buffer (50 mM sodium phosphate, 300 mM sodium chloride, 150 mM imidazole, pH 7.0) was added to the residue. The supernatant was recovered by centrifugation. Then, 100 µL of an elution buffer was added thereto again. This series of operation was performed three times, and the obtained eluates were mixed. The eluate mixture was placed in a dialysis tube (Sanko Junyaku Co., Ltd.) washed by boiling, and dialyzed against a 50 mM HEPES-NaOH buffer (pH 7.5). The solution thus dialyzed was recovered and used as a purified enzyme solution of each His-tagged bacterium-derived Nampt.

### (4) NMN synthesis reaction and analysis of NMN

NMN synthesis reaction and analysis were performed using the cell-free extracts of each enzyme other than Nampt obtained in (2), and the purified enzyme of Nampt. The purified enzymes of bacterium-derived Nampt used were three purified His-tagged Nampt enzymes prepared in (3). The purified enzyme of human-derived Nampt (HsNampt-His) used was a commercially available purified enzyme of His-tagged human-derived Nampt (CY-E1251, MBL). The procedures were performed in the same way as in Examples 1(5) and 1(6) except that: each reaction solution was prepared according to the composition shown in Table 12; and the reaction solution was sampled 12 hours after the start of the reaction.

**Table 12**

| Reaction solution composition (final concentration) | | Reaction sample | | | | |
|---|---|---|---|---|---|---|
| | | No. 5-1 | No. 5-2 | No. 5-3 | No. 5-4 | No. 5-5 |
| | | without Nampt | DrNampt-His | HdNampt-His | SoNampt-His | HsNampt-His |
| HEPES-NaOH (pH7.5) [mM] | | 50 | 50 | 50 | 50 | 50 |
| Nicotinamide [mM] | | 20 | 20 | 20 | 20 | 20 |
| Ribose [mM] | | 100 | 100 | 100 | 100 | 100 |
| Magnesium chloride [mM] | | 10 | 10 | 10 | 10 | 10 |
| Polyphosphate [mM] | | 3 | 3 | 3 | 3 | 3 |
| Adenosine triphosphate [mM] | | 0.1 | 0.1 | 0.1 | 0.1 | 0.1 |
| Potassium phosphate buffer [mM] | | 5 | 5 | 5 | 5 | 5 |
| Dithiothreitol [mM] | | 1 | 1 | 1 | 1 | 1 |
| Ammonium sulfate [ mM ] | | 20 | 20 | 20 | 20 | 20 |
| Cell-free extracts | ScRbk [g/L] | 0.03 | 0.03 | 0.03 | 0.03 | 0.03 |
| | BsPrsN120S [g/L] | 0.2 | 0.2 | 0.2 | 0.2 | 0.2 |
| | DrPpk [g/L] | 1.4 | 1.4 | 1.4 | 1.4 | 1.4 |
| Purified enzyme | Each Nampt [g/L] | - | 0.06 | 0.06 | 0.06 | 0.06 |

### (5) Experimental results

The experimental results are shown in Figure 5. In the case of using bacterium-derived Nampt (HdNampt-His, DrNampt-His and SoNampt-His), 1.4 mM to 2.4 mM NMN was generated. The number of moles of the added ATP (0.01 µmol) was 0.042 to 0.071 equivalents of the number of moles of the generated NMN (0.14 to 0.24 µmol). On the other hand, in the case of using human-derived Nampt (HsNampt-His), the amount of NMN generated was 0.6 mM. The number of moles of the added ATP (0.01 µmol) was 0.17 equivalents of the number of moles of the generated NMN (0.06 µmol). These results demonstrated that NMN may be synthesized using any of bacterium-derived Nampt and human-derived Nampt, while NMN can be efficiently synthesized, particularly, by using bacterium-derived Nampt.

### [Example 6] <NMN synthesis from R5P through use of ATP regeneration system>

### (1) Preparation of recombinant, culture and preparation of cell-free extracts

In this Example, cell-free extracts of each enzyme were prepared by the same operation as in Examples 1(2) to 1(4).

### (2) NMN synthesis reaction and analysis of NMN

NMN synthesis reaction and analysis were performed using the cell-free extracts obtained in (1). The amount of the reaction solution was set to 100 µL, and the procedures were performed in the same way as in Examples 1(5) and 1(6) except that: each reaction solution was prepared according to the composition shown in Table 13 (No. 6-2); and the reaction solution was sampled immediately after the start of the reaction (0 hours) and 6 hours later.

**Table 13**

| Reaction solution composition (final concentration) | | Reaction sample | |
|---|---|---|---|
| | | No. 6-1 | No. 6-2 |
| | | without Ppk | with Ppk |
| HEPES-NaOH (pH7.5) [mM] | | 50 | 50 |
| Nicotinamide [mM] | | 10 | 10 |
| R5P [mM] | | 10 | 10 |
| Magnesium chloride [mM] | | 10 | 10 |
| Polyphosphate [mM] | | 5 | 5 |
| Adenosine triphosphate [mM] | | 0.1 | 0.1 |
| Potassium phosphate buffer [mM] | | 5 | 5 |
| Ammonium sulfate [mM] | | 20 | 20 |
| Cell-free extracts | BsPrs [g/L] | 0.6 | 0.6 |
| | HdNampt [g/L] | 0.2 | 0.2 |
| | DrPpk [g/L] | - | 1.4 |

### (3) Experimental results

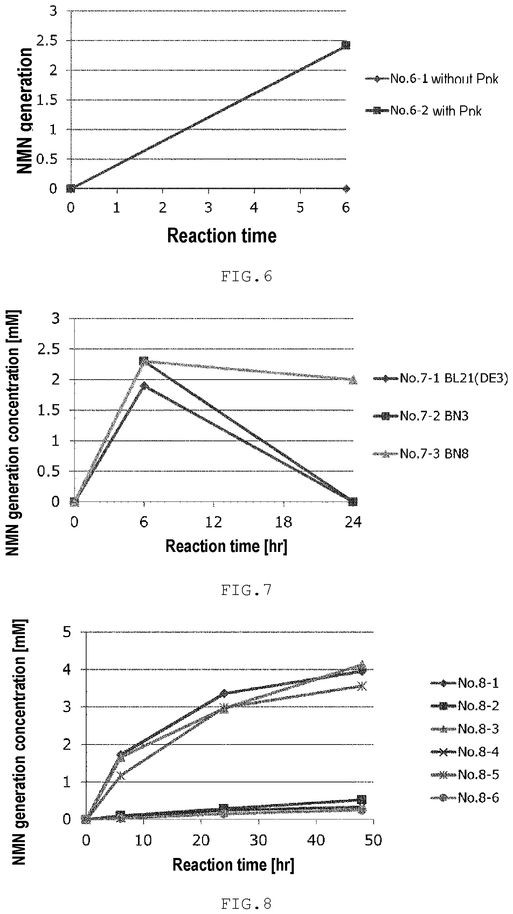

The experimental results are shown in Figure 6. The generation of 2.4 mM NMN was observed in sample No. 6-2 supplemented with Ppk. The number of moles of the added ATP (0.01 µmol) was 0.042 equivalents of the number of moles of the generated NMN (0.24 µmol). These results demonstrated that NMN can be efficiently synthesized from R5P by the coexistence of the ATP-regenerating enzyme Ppk.

### [Comparative Example 2] <NMN synthesis from R5P without use of ATP regeneration system>

### (1) Preparation of recombinant, culture and preparation of cell-free extracts

In this Comparative Example, cell-free extracts of each enzyme were prepared by the same operation as in Examples 1(2) to 1(4).

### (2) NMN synthesis reaction and analysis of NMN

NMN synthesis reaction and analysis were performed using the cell-free extracts obtained in (1). The amount of the reaction solution was set to 100 µL, and the procedures were performed in the same way as in Examples 1(5) and 1(6) except that: each reaction solution was prepared according to the composition shown in Table 13 (No. 6-1); and the reaction solution was sampled immediately after the start of the reaction (0 hours) and 6 hours later.

### (3) Experimental results

The experimental results are shown in Figure 6. NMN was generated at a level equal to or lower than the detection limit in sample No. 6-1 supplemented with no Ppk.

### [Example 7] <NMN synthesis using cell-free extracts prepared from host having unnecessary gene disrupted>

### (1) Preparation of host having unnecessary gene disrupted

In order to suppress the degradation or side reaction of the reactant (substrate) NAM and the product NMN, the hosts of Table 14 were prepared which has disruption of a gene encoding each enzyme responsible for the degradation or the side reaction.

**Table 14**

| Host name | Enzyme responsible for degradation or side reaction | Disrupted gene |
|---|---|---|
| BN1 | (a) EC 3. 1. 3. 5 (5'-nucleotidase): NMN degradation | ushA |
| BN3 | (a) EC 3. 1. 3. 5 (5'-nucleotidase): NMN degradation | ushA |
| | (b) EC 3. 5. 1. 19 (nicotinamidase): NAM degradation | pncA |
| BN6 | (a) EC 3. 1. 3. 5 (5'-nucleotidase): NMN degradation | ushA |
| | (b) EC 3. 5. 1. 19 (nicotinamidase):NAM degradation | pncA |
| | (d) EC 3. 5. 1. 42 (nicotinamide mononucleotide deamidase) NMN degradation | pncC |
| BN8 | (a) EC 3. 1. 3. 5 (5'-nucleotidase): NMN degradation | ushA |
| | (b) EC 3. 5. 1. 19 (nicotinamidase): NAM degradation | pncA |
| | (d) EC 3. 5. 1. 42 (nicotinamide mononucleotide deamidase) NMN degradation | pncC |
| | (a) EC 3. 1. 3. 5 (5'-nucleotidase): NMN degradation | yrfG |

The hosts having each unnecessary gene disrupted were prepared using TargeTron Gene Knockout System (Sigma-Aldrich Co. LLC), basically, according to the attached protocol.

### Preparation of BN1 strain

First, a BN1 strain having ushA gene disrupted was prepared as described below with *Escherichia coli* BL21 (DE3) as a host. PCR was first performed according to the reaction solution composition shown in Table 15 and the reaction conditions shown in Table 16 using each primer shown in SEQ ID NO: 31 (IBS primer), SEQ ID NO: 32 (EBS1d primer) and SEQ ID NO: 33 (EBS2 primer), and EBS Universal primer attached to TargeTron Gene Knockout System.

**Table 15**

| Component | Added amount |
|---|---|
| Sterilized water | 23 µL |
| Primer mixed solution (5 µM IBS primer, 5 µM EBS1d primer, 1 µM EBS2 primer, 1 µM EBS Universal primer) | 1 µL |
| Template for intron PCR (attached to kit) | 1 µL |
| JumpStart REDTaq ReadyMix (attached to kit) | 25 µL |

**Table 16**

| Temperature | Time | The number of cycles |
|---|---|---|
| 94°C | 30 sec | - |
| 94°C | 15 sec | 30 |
| 55°C | 30 sec | |
| 72°C | 30 sec | |
| 72°C | 2 min | - |
| 4°C | - | - |

After the completion of the PCR reaction, 3 µL of the reaction solution was electrophoresed on 4% agarose gel to confirm that a DNA fragment of approximately 350 bp was amplified. The remaining PCR reaction solution was purified using QIAquick PCR Purification Kit (Qiagen N.V.). 2 µL of 103 Restriction Enzyme Buffer (attached to the kit), 1 µL of HindIII, 1 µL of BsrGI and 8 µL of sterilized water were added to 8 µL of the purified PCR reaction solution. The PCR product was digested at 37°C for 30 minutes and subsequently at 60°C for 30 minutes and then treated at 80°C for 10 minutes. 3 µL of the obtained digested product was heat-treated at 60°C for 30 seconds and then cooled on ice for 1 minute. 1 µL of pACD4K-C Linear Vector (attached to the kit) and 4 µL of DNA Ligation Kit <Mighty Mix> (Takara Bio Inc.) were added thereto and mixed, followed by ligation reaction at 16°C for 1 hour. 5 µL of the ligation reaction solution was mixed with 50 µL of *Escherichia coli* JM109 competent cells (Takara Bio Inc.) and left standing on ice for 30 minutes. The cells were incubated at 42°C for 45 seconds and then left standing on ice again for 5 minutes. 500 µL of SOC medium was added thereto, and the cells were shake-cultured at 200 rpm at 37°C for 1 hour and then spread in an appropriate amount over LB agar medium (containing 25 µg/mL chloramphenicol). After culture overnight at 37°C, the grown colony was inoculated to LB liquid medium (containing 25 µg/mL chloramphenicol) and shake-cultured at 200 rpm at 37°C for 16 hours. The culture solution was centrifuged to recover microbial cells. A plasmid was extracted therefrom using QIAprep Spin Miniprep Kit (Qiagen N.V.) according to the attached protocol. The obtained plasmid DNA was digested with HindIII and then electrophoresed on 1% agarose gel. A plasmid confirmed to provide a band of approximately 7.7 kbp was used as pACD4K-C-ushA.

Subsequently, in order to prepare a plasmid for ushA gene disruption free from kanamycin resistance gene, PCR reaction was performed with pACD4K-C-ushA as a template according to the reaction solution composition shown in Table 17 and the reaction conditions shown in Table 18 using primers shown in SEQ ID NO: 34 (pACD4K-C-dKm-F2) and SEQ ID NO: 35 (pACD4K-C-dKm-R). The primer shown in SEQ ID NO: 34 was 5'-terminally modified by phosphorylation and used.

**Table 17**

| Component | Added amount |
|---|---|
| Sterilized water | 13 µL |
| PrimeSTAR Max Premix | 25 µL |
| pACD4K-C-dKm-F2 (2 µM) | 5 µL |
| pACD4K-C-dKm-R (2 µM) | 5 µL |
| pACD4K-C-ushA (0.5 ng/µL) | 2 µL |

**Table 18**

| Temperature | Time | The number of cycles |
|---|---|---|
| 98°C | 30 sec | - |
| 98°C | 10 sec | 30 |
| 55°C | 15 sec | |
| 72°C | 30 sec | |
| 72°C | 2 min | - |
| 4°C | - | - |

After the completion of the PCR reaction, 1 µL of DpnI (Takara Bio Inc.) was added to the reaction solution and reacted at 37°C for 1 hour. 2 µL of DNA Ligation Kit <Mighty Mix> (Takara Bio Inc.) was added to 2 µL of the reaction solution purified with QIAquick PCR Purification Kit (Qiagen N.V.), and mixed, followed by ligation reaction at 16°C for 1 hour. The ligation reaction solution was mixed with 40 µL of *Escherichia coli* JM109 competent cells (Takara Bio Inc.) and left standing on ice for 30 minutes. The cells were incubated at 42°C for 45 seconds and then left standing on ice again for 5 minutes. 500 µL of SOC medium was added thereto, and the cells were shake-cultured at 200 rpm at 37°C for 1 hour and then spread in an appropriate amount over LB agar medium (containing 25 µg/mL chloramphenicol). After culture overnight at 37°C, the grown colony was inoculated to LB liquid medium (containing 25 µg/mL chloramphenicol) and shake-cultured at 200 rpm at 37°C for 16 hours. The culture solution was centrifuged to recover microbial cells. A plasmid was extracted therefrom using QIAprep Spin Miniprep Kit (Qiagen N.V.) according to the attached protocol. The obtained plasmid DNA was digested with MluI and then electrophoresed on 1% agarose gel. A plasmid confirmed to provide a band of approximately 6.3 kbp was used as pACD4K-C-ushAΔKm.

1 µL of the pACD4K-C-ushAΔKm plasmid solution was added to 50 µL of *Escherichia coli* BL21 (DE3) competent cells (BioDynamics Laboratory Inc.) and left standing on ice for 10 minutes. The cells were heat-shocked at 42°C for 45 seconds and left standing on ice again for 5 minutes. 450 µL of SOC medium brought to room temperature was added thereto, and the cells were shake-cultured at 200 rpm at 37°C for 1 hour. 100 µL of the culture solution thus shaken was added to 3 mL of LB liquid medium (containing 1% glucose and 25 µg/ml chloramphenicol) and shake-cultured overnight at 200 rpm at 37°C. 40 µL of the obtained culture solution was added to 2 mL of LB liquid medium (containing 1% glucose and 25 µg/ml chloramphenicol), and the culture was continued at 37°C until OD600 became approximately 0.2. After OD600 became approximately 0.2, 10 µL of a 100 mM IPTG solution was added to the culture solution, which was then shake-cultured at 200 rpm at 30°C for 30 minutes. Then, the culture solution was centrifuged at the maximum speed for 1 minute using a microcentrifuge, and microbial cells were resuspended in 1 mL of LB liquid medium (containing 1% glucose and no chloramphenicol). After shake culture at 20 rpm at 30°C for 1 hour, 100 µL of the culture solution was spread over LB agar plate coated in advance with 100 µL of a 100 mM IPTG solution, and cultured at 30°C for 3 days. A plurality of colonies that appeared were subjected to colony PCR using a forward primer (SEQ ID NO: 36) and a reverse primer (SEQ ID NO: 37). The reaction solution composition was as shown in Table 19, and the reaction conditions were as shown in Table 18. A band of approximately 1.1 kbp was amplified for the original host (BL21 (DE3)), whereas a clone was obtained in which a band of approximately 1.8 kbp was amplified. This clone was used as a BN1 strain, a host having the ushA gene disrupted. Competent cells of the BN1 strain were prepared using E. coli Transformation Kit, Mix & Go (Zymo Research Corp.) according to the attached protocol.

**Table 19**

| Component | Added amount |
|---|---|
| Sterilized water | 15 µL |
| PrimeSTAR Max Premix | 25 µL |
| Forward primer (2 µM) | 5 µL |
| Reverse primer (2 µM) | 5 µL |
| Colony | - |

### Preparation of BN3 strain

Subsequently, a BN3 strain having pncA gene disrupted was prepared as described below with the BN1 strain as a host. PCR was performed according to the reaction solution composition shown in Table 15 and the reaction conditions shown in Table 16 using each primer shown in SEQ ID NO: 38 (IBS primer), SEQ ID NO: 39 (EBS1d primer) and SEQ ID NO: 40 (EBS2 primer), and EBS Universal primer attached to TargeTron Gene Knockout System. Purification of the PCR reaction solution, restriction enzyme treatment with HindIII and BsrGI, ligation reaction with vector pACD4K-C, transformation of *Escherichia coli* JM109 and plasmid extraction were performed in the same way as in the preparation of the BN1 strain. The obtained plasmid DNA was digested with HindIII and then electrophoresed on 1% agarose gel. A plasmid confirmed to provide a band of approximately 7.7 kbp was used as pACD4K-C-pncA.

A plasmid for pncA gene disruption free from kanamycin resistance gene was prepared. This preparation was performed by the same operation as in the preparation of pACD4K-C-ushAΔKm except that pACD4K-C-pncA was used as a template in PCR reaction. The obtained plasmid was used as pACD4K-C-pncAΔKm.

A BN3 strain having pncA gene disrupted was prepared with the BN1 strain as a host. The BN3 strain was prepared by the same operation as in the preparation of the BN1 strain mentioned above except that: the gene disruption plasmid used was pACD4K-C-pncAΔKm; and the competent cells used were BN1 strain competent cells. A plurality of colonies that appeared were subjected to colony PCR using a forward primer (SEQ ID NO: 41) and a reverse primer (SEQ ID NO: 42). The reaction solution composition was as shown in Table 19, and the reaction conditions were as shown in Table 18.

A band of approximately 2.2 kbp was amplified for the original host (BL21 (DE3)), whereas a clone was obtained in which a band of approximately 2.9 kbp was amplified. This clone was used as a BN3 strain, a host having the ushA gene and the pncA gene disrupted. Competent cells of the BN3 strain were prepared using E. coli Transformation Kit, Mix & Go (Zymo Research Corp.) according to the attached protocol.

### Preparation of BN6 strain

Subsequently, a BN6 strain having pncC gene disrupted was prepared as described below with the BN3 strain as a host. PCR was performed according to the reaction solution composition shown in Table 15 and the reaction conditions shown in Table 16 using each primer shown in SEQ ID NO: 43 (IBS primer), SEQ ID NO: 44 (EBS1d primer) and SEQ ID NO: 45 (EBS2 primer), and EBS Universal primer attached to TargeTron Gene Knockout System. Purification of the PCR reaction solution, restriction enzyme treatment with HindIII and BsrGI, ligation reaction with vector pACD4K-C, transformation of *Escherichia coli* JM109 and plasmid extraction were performed in the same way as in the preparation of the BN1 strain. The obtained plasmid DNA was digested with HindIII and then electrophoresed on 1% agarose gel. A plasmid confirmed to provide a band of approximately 7.7 kbp was used as pACD4K-C-pncC.

A plasmid for pncC gene disruption free from kanamycin resistance gene was prepared. This preparation was performed by the same operation as in the preparation of pACD4K-C-ushAΔKm except that pACD4K-C-pncC was used as a template in PCR reaction. The obtained plasmid was used as pACD4K-C-pncAΔKm.

A BN6 strain having pncC gene disrupted was prepared with the BN3 strain as a host. The BN6 strain was prepared by the same operation as in the preparation of the BN1 strain mentioned above except that the gene disruption plasmid used was pACD4K-C-pncCΔKm. A plurality of colonies that appeared were subjected to colony PCR using a forward primer (SEQ ID NO: 46) and a reverse primer (SEQ ID NO: 47). The reaction solution composition was as shown in Table 19, and the reaction conditions were as shown in Table 18.

A band of approximately 0.5 kbp was amplified for the original host (BL21 (DE3)), whereas a clone was obtained in which a band of approximately 1.2 kbp was amplified. This clone was used as a BN6 strain, a host having the ushA gene, the pncA gene and the pncC gene disrupted. Competent cells of the BN6 strain were prepared using E. coli Transformation Kit, Mix & Go (Zymo Research Corp.) according to the attached protocol.

### Preparation of BN8 strain

Subsequently, a BN8 strain having yrfG gene disrupted was prepared as described below with the BN6 strain as a host. PCR was performed according to the reaction solution composition shown in Table 15 and the reaction conditions shown in Table 16 using each primer shown in SEQ ID NO: 48 (IBS primer), SEQ ID NO: 49 (EBS1d primer) and SEQ ID NO: 50 (EBS2 primer), and EBS Universal primer attached to TargeTron Gene Knockout System. Purification of the PCR reaction solution, restriction enzyme treatment with HindIII and BsrGI, ligation reaction with vector pACD4K-C, transformation of *Escherichia coli* JM109 and plasmid extraction were performed in the same way as in the preparation of the BN1 strain. The obtained plasmid DNA was digested with HindIII and then electrophoresed on 1% agarose gel. A plasmid confirmed to provide a band of approximately 7.7 kbp was used as pACD4K-C-yrfG.

A plasmid for yrfG gene disruption free from kanamycin resistance gene was prepared. This preparation was performed by the same operation as in the preparation of pACD4K-C-ushAΔKm except that pACD4K-C-yrfG was used as a template in PCR reaction. The obtained plasmid was used as pACD4K-C-yrfGΔKm.

A BN8 strain having yrfG gene disrupted was prepared with the BN6 strain as a host. The BN8 strain was prepared by the same operation as in the preparation of the BN1 strain mentioned above except that: the gene disruption plasmid used was pACD4K-C-yrfGΔKm; and the competent cells used were BN6 strain competent cells. A plurality of colonies that appeared were subjected to colony PCR using a forward primer (SEQ ID NO: 51) and a reverse primer (SEQ ID NO: 52). The reaction solution composition was as shown in Table 19, and the reaction conditions were as shown in Table 18.

A band of approximately 0.4 kbp was amplified for the original host (BL21 (DE3)), whereas a clone was obtained in which a band of approximately 1.1 kbp was amplified. This clone was used as a BN8 strain, a host having the ushA gene, the pncA gene, the pncC gene and the yrfG gene disrupted. Competent cells of the BN8 strain were prepared using E. coli Transformation Kit, Mix & Go (Zymo Research Corp.) according to the attached protocol.

### (2) Preparation of recombinant, culture and preparation of cell-free extracts

In this Example, cell-free extracts of each enzyme were prepared by the same operation as in Examples 1(2) to 1(4) except that: BsPrsN120S described in Example 3 was used as Prs; and the BN3 strain and the BN8 strain described in (1) of this Example were used as hosts for enzyme expression.

### (3) NMN synthesis reaction and analysis of NMN

NMN synthesis reaction and analysis were performed using the cell-free extracts obtained in (1). The procedures were performed in the same way as in Examples 1(5) and 1(6) except that: each reaction solution was prepared according to the composition shown in Table 20; and the reaction solution was sampled immediately after the start of the reaction (0 hours), 6 hours later and 24 hours later.

**Table 20**

| Reaction solution composition (final concentration) | | Reaction sample | | |
|---|---|---|---|---|
| | | No. 7 -1 | No. 7-2 | No. 7-3 |
| | | BL21 (DE3) | BN3 | BN8 |
| HEPES-NaOH (pH7.5) [mM] | | 50 | 50 | 50 |
| Nicotinamide [mM] | | 10 | 10 | 10 |
| Ribose [mM] | | 10 | 10 | 10 |
| Magnesium chloride [mM] | | 10 | 10 | 10 |
| Polyphosphate [mM] | | 5 | 5 | 5 |
| Adenosine triphosphate | | 0.1 | 0.1 | 0.1 |
| Potassium phosphate buffer [mM] | | 5 | 5 | 5 |
| Ammonium sulfate [mM] | | 20 | 20 | 20 |
| Cell-free extracts | ScRbk [g/L] | 0.04 | 0.04 | 0.04 |
| | BsPrs [g/L] | 0.6 | 0.6 | 0.6 |
| | HdNampt [g/L] | 0.2 | 0,2 | 0.2 |
| | DrPpk [g/L] | 1.4 | 1.4 | 1.4 |

### (4) Experimental results

The experimental results are shown in Figure 7. In the case of using cell-free extracts prepared with usual BL21 (DE3) as a host (sample No. 7-1), the generation of 1.9 mM NMN was observed at 6 hours after the start of the reaction. The number of moles of the added ATP (0.01 µmol) was 0.052 equivalents of the number of moles of the generated NMN (0.19 (µmol). On the other hand, in the case of using cell-free extracts prepared with the BN3 strain having ushA gene and pncA gene disrupted as a host (sample No. 7-2), the generation of 2.3 mM NMN was observed. The number of moles of the added ATP (0.01 µmol) was 0.043 equivalents of the number of moles of the generated NMN (0.23 (µmol). In the case of using cell-free extracts prepared with the BN8 strain having ushA gene, pncA gene, pncC gene and yrfG gene disrupted as a host (sample No. 7-3), the generation of 2.3 mM NMN was also observed. The number of moles of the added ATP (0.01 µmol) was 0.043 equivalents of the number of moles of the generated NMN (0.23 (µmol). These results demonstrated that at the stage where the generation of NMN proceeds, the amount of NMN produced is higher using cell-free extracts prepared with the BN3 strain or the BN8 strain as a host than using cell-free extracts prepared with BL21 (DE3) as a host.

In the case of using cell-free extracts prepared with usual BL21 (DE3) or the BN3 strain as a host (sample No. 7-1 and No. 7-2), NMN was not detectable at 24 hours after the start of the reaction. In the case of using cell-free extracts prepared with the BN8 strain as a host (sample No. 7-3), 2.0 mM NMN remained. These results demonstrated that at a stage after a given amount of NMN is generated, the degradation of the generated NMN is more significantly suppressed using cell-free extracts prepared with BN8 as a host than using cell-free extracts prepared with BL21 (DE3) or the BN3 strain as a host. However, the generated NMN can be appropriately obtained even using cell-free extracts derived from any host, provided that the step of appropriately recovering the generated NMN is performed at a point in time (e.g., 6 hours after the start of the reaction) before the degradation of NMN proceeds.

### [Example 8] <NMN synthesis with purified enzyme using PPase>

In this Example, the predetermined enzymes used were the following enzymes.
Nampt: His-tagged *Haemophilus ducreyi*-derived (AAR87771) Nampt (HdNampt-His)
Prs: His-tagged Asn120Ser mutant of *Bacillus* subtilis-derived (BAA05286) Prs (BsPrsN120S-His)
Ppk (Ppk2 class 3): His-tagged *Deinococcus radiodurans*-derived (NP_293858) Ppk (DrPpk-His)
Rbk: His-tagged *Saccharomyces cerevisiae-derived* (P25332) Rbk (ScRbk-His)

### (1) Preparation of His-tagged Prs, Ppk and Rbk expression plasmids

Expression plasmids for His-tagged proteins of Prs, Ppk and Rbk were prepared as described below. Mutagenesis PCR reaction for His-tagging each enzyme was performed with pEBsPrsN120S prepared in Example 3 and pEDrPpk and pEScRbk prepared in Example 1 as templates. Template plasmid names, primer names for mutagenesis, SEQ ID NOs representing the nucleotide sequences of the primers, and His-tagged enzyme expression plasmid names are shown in Table 21.

**Table 21**

| Template plasmid | Primer for mutagenesis | | His-tagged Nampt expression plasmid |
|---|---|---|---|
| pEBsPrsN120S | BsPrs-His F | SEQ ID NO: 53 | pEBsPrsN120S-His |
| | BsPrs-His R | SEQ ID NO: 54 | |
| pEDrPpk | DrPpk-His F | SEQ ID NO: 55 | pEDrPpk-His |
| | DrPpk-His R | SEQ ID NO: 56 | |
| pEScRbk | ScRbk-His F | SEQ ID NO: 57 | pEScRbk-His |
| | ScRbk-His R | SEQ ID NO: 58 | |

The preparation of each His-tagged enzyme expression plasmid from mutagenesis PCR reaction to sequencing was performed by the same operation as in Example 3(1) except that various template plasmids and mutagenesis primers shown in Table 21 were used. A correctly His-tagged plasmid was used as each plasmid shown in Table 21.

### (2) Preparation of recombinant, culture and preparation of cell-free extracts

Preparation of a recombinant, culture and preparation of cell-free extracts were performed by the same operation as in Example 4(1) using each His-tagged enzyme expression plasmid obtained above in (1). In this respect, a 50 mM HEPES-NaOH buffer (pH 8.0) was used as a buffer for suspending microbial cells except for BsPrsN120S-His. However, a 50 mM potassium phosphate buffer (pH 7.5) was used as to BsPrsN120S-His.

### (3) Preparation of purified enzyme

Each His-tagged enzyme was purified by immobilized metal affinity chromatography in the same way as in Example 5(3) using the cell-free extracts containing the His-tagged enzyme, prepared in (2). However, the solution of BsPrsN120S-His was dialyzed against a 50 mM potassium phosphate buffer (pH 7.5).

### (4) NMN synthesis reaction and analysis of NMN

NMN synthesis reaction and analysis were performed in the presence or absence of PPase (yeast-derived, Sigma-Aldrich Co. LLC, product No. 10108987001) using various purified enzymes obtained in (3). The procedures were performed in the same way as in Examples 1(5) and 1(6) except that: each reaction solution was prepared according to the composition shown in Table 22; and the reaction solution was sampled immediately after the start of the reaction (0 hours), 6 hours later, 24 hours later and 48 hours later.

**Table 22**

| Reaction solution composition (final concentration) | | Reaction sample | | | | | |
|---|---|---|---|---|---|---|---|
| | | No. 8-1 | No. 8-2 | No. 8-3 | No. 8-4 | No. 8-5 | No. 8-6 |
| | | 3rd reaction | | 2nd + 3rd reactions | | 1st + 2nd + 3rd reactions | |
| | | with PPase | without PPase | with PPase | without PPase | with PPase | without PPase |
| HEPES-NaOH (pH7.5) [mM] | | 50 | 50 | 50 | 50 | 50 | 50 |
| Nicotinamide [mM] | | 10 | 10 | 10 | 10 | 10 | 10 |
| Ribose [mM] | | 0 | 0 | 0 | 0 | 10 | 10 |
| R5P [mM] | | 0 | 0 | 10 | 10 | 0 | 0 |
| PRPP [mM] | | 10 | 10 | 0 | 0 | 0 | 0 |
| Magnesium chloride [mM] | | 10 | 10 | 10 | 10 | 10 | 10 |
| Polyphosphate [mM] | | 5 | 5 | 5 | 5 | 5 | 5 |
| Adenosine triphosphate [mM] | | 1 | 1 | 1 | 1 | 1 | 1 |
| Potassium phosphate buffer [mM] | | 5 | 5 | 5 | 5 | 5 | 5 |
| Ammonium sulfate [ mM ] | | 20 | 20 | 20 | 20 | 20 | 20 |
| Cell-free extracts | ScRbk [g/L] | 0 | 0 | 0 | 0 | 0.02 | 0.02 |
| | BsPrs [g/L] | 0 | 0 | 0.07 | 0.07 | 0.07 | 0.07 |
| | HdNampt [g/L] | 0.1 | 0.1 | 0.1 | 0.1 | 0.1 | 0.1 |
| | DrPpk [g/L] | 1.4 | 1.4 | 1.4 | 1.4 | 1.4 | 1.4 |
| PPase [g/L] | | 0.01 | 0 | 0.01 | 0 | 0.01 | 0 |

### (5) Experimental results

The experimental results are shown in Figure 8. In the case of adding PPase (sample No. 8-1), the third reaction alone generated 4.0 mM NMN for 48 hours of the reaction. The number of moles of the added ATP (0.1 µmol) was 0.25 equivalents of the number of moles of the generated NMN (0.40 (µmol). On the other hand, in the case of adding no PPase (sample No. 8-2), 0.52 mM NMN was generated. The number of moles of the added ATP (0.1 µmol) was 1.9 equivalents of the number of moles of the generated NMN (0.052 (µmol). In the case of adding PPase (sample No. 8-3), the second reaction + the third reaction generated 4.1 mM NMN for 48 hours of the reaction. The number of moles of the added ATP (0.1 µmol) was 0.24 equivalents of the number of moles of the generated NMN (0.41 (µmol). On the other hand, in the case of adding no PPase (sample No. 8-4), 0.34 mM NMN was generated. The number of moles of the added ATP (0.1 µmol) was 2.9 equivalents of the number of moles of the generated NMN (0.034 (µmol). In the case of adding PPase (sample No. 8-5), the first reaction + the second reaction + the third reaction generated 3.6 mM NMN for 48 hours of the reaction. The number of moles of the added ATP (0.1 µmol) was 0.28 equivalents of the number of moles of the generated NMN (0.36 (µmol). On the other hand, in the case of adding no PPase (sample No. 8-6), 0.25 mM NMN was generated. The number of moles of the added ATP (0.1 µmol) was 4.0 equivalents of the number of moles of the generated NMN (0.025 (µmol). These results demonstrated that NMN can be efficiently synthesized by adding PPase to the purified enzyme reaction system.

### Free Text of Sequence Listing

SEQ ID NO: 15: primer for mutagenesis of Prs mutant BsPrsN120S (forward)
SEQ ID NO: 16: primer for mutagenesis of Prs mutant BsPrsN120S (reverse)
SEQ ID NO: 17: primer for mutagenesis of Prs mutant BsPrsL135I (forward)
SEQ ID NO: 18: primer for mutagenesis of Prs mutant BsPrsL135I (reverse)
SEQ ID NO: 19: primer T7-PP
SEQ ID NO: 20: primer T7-TP
SEQ ID NO: 25: primer for mutagenesis of His-tagged Nampt expression plasmid pEHdNampt-His (forward)
SEQ ID NO: 26: primer for mutagenesis of His-tagged Nampt expression plasmid pEHdNampt-His (reverse)
SEQ ID NO: 27: primer for mutagenesis of His-tagged Nampt expression plasmid pEDrNampt-His (forward)
SEQ ID NO: 28: primer for mutagenesis of His-tagged Nampt expression plasmid pEDrNampt-His (reverse)
SEQ ID NO: 29: primer for mutagenesis of His-tagged Nampt expression plasmid pESoNampt-His (forward)
SEQ ID NO: 30: primer for mutagenesis of His-tagged Nampt expression plasmid pESoNampt-His (reverse)
SEQ ID NO: 31: IBS primer for ushA
SEQ ID NO: 32: EBSld primer for ushA
SEQ ID NO: 33: EBS2 primer for ushA
SEQ ID NO: 34: primer for preparation of pACD4K-C-ushAΔKm (forward)
SEQ ID NO: 35: primer for preparation of pACD4K-C-ushAΔKm (reverse)
SEQ ID NO: 36: primer for detection of ushA gene disruption (forward)
SEQ ID NO: 37: primer for detection of ushA gene disruption (reverse)
SEQ ID NO: 38: IBS primer for pncA
SEQ ID NO: 39: EBSld primer for pncA
SEQ ID NO: 40: EBS2 primer for pncA
SEQ ID NO: 41: primer for detection of pncA gene disruption (forward)
SEQ ID NO: 42: primer for detection of pncA gene disruption (reverse)
SEQ ID NO: 43: IBS primer for pncC
SEQ ID NO: 44: EBSld primer for pncC
SEQ ID NO: 45: EBS2 primer for pncC
SEQ ID NO: 46: primer for detection of pncC gene disruption (forward)
SEQ ID NO: 47: primer for detection of pncC gene disruption (reverse)
SEQ ID NO: 48: IBS primer for yrfG
SEQ ID NO: 49: EBSld primer for yrfG
SEQ ID NO: 50: EBS2 primer for yrfG
SEQ ID NO: 51: primer for detection of yrfG gene disruption (forward)
SEQ ID NO: 52: primer for detection of yrfG gene disruption (reverse)
SEQ ID NO: 53: primer for mutagenesis of His-tagged Nampt expression plasmid pEBsPrsN120S-His (forward)
SEQ ID NO: 54: primer for mutagenesis of His-tagged Nampt expression plasmid pEBsPrsN120S-His (reverse)
SEQ ID NO: 55: primer for mutagenesis of His-tagged Nampt expression plasmid pEDrPpk-His (forward)
SEQ ID NO: 56: primer for mutagenesis of His-tagged Nampt expression plasmid pEDrPpk-His (reverse)
SEQ ID NO: 57: primer for mutagenesis of His-tagged Nampt expression plasmid pEScRbk-His (forward)
SEQ ID NO: 58: primer for mutagenesis of His-tagged Nampt expression plasmid pEScRbk-His (reverse)

## Claims

1. A method for producing nicotinamide mononucleotide (NMN), comprising the step of bringing a transformant with enhanced expression of three enzymes nicotinamide phosphoribosyltransferase (Nampt), phosphoribosyl pyrophosphate synthetase (Prs) and polyphosphate kinase (Ppk), a cell-free protein synthesis reaction solution having the three enzymes expressed, or a treated product thereof into contact with ribose-5-phosphate (R5P), nicotinamide (NAM), ATP and polyphosphate.

2. The method for producing NMN according to claim 1, further comprising the step of bringing a transformant with enhanced expression of two enzymes ribokinase (Rbk) and polyphosphate kinase (Ppk), a cell-free protein synthesis reaction solution having the two enzymes expressed, or a treated product thereof into contact with ribose, ATP and polyphosphate to produce the R5P.

3. The method for producing NMN according to claim 1 or 2, wherein the method is performed under conditions where the transformant does not substantially proliferate.

4. The method for producing NMN according to any one of claims 1 to 3, wherein the Nampt is bacterium-derived.

5. The method for producing NMN according to any one of claims 1 to 4, wherein the Ppk belongs to the polyphosphate kinase type 2 family.

6. The method for producing NMN according to any one of claims 1 to 5, wherein a host of the transformant is *Escherichia coli,* a bacterium of the genus *Corynebacterium,* a bacterium of the genus *Rhodococcus,* or a yeast.

7. A transformant with enhanced expression of three enzymes nicotinamide phosphoribosyltransferase (Nampt), phosphoribosyl pyrophosphate synthetase (Prs) and polyphosphate kinase (Ppk).

8. A transformant with enhanced expression of two enzymes ribokinase (Rbk) and polyphosphate kinase (Ppk).

9. A method for producing nicotinamide mononucleotide (NMN), comprising the step of bringing a transformant with enhanced expression of nicotinamide phosphoribosyltransferase (Nampt), a cell-free protein synthesis reaction solution having the enzyme expressed, or a treated product thereof into contact with nicotinamide (NAM) and phosphoribosyl pyrophosphate (PRPP) in the presence of pyrophosphatase (PPase).

10. The method for producing NMN according to claim 9, wherein the method is performed under conditions where the transformant does not substantially proliferate.

11. The method for producing NMN according to claim 9 or 10, wherein the treated product is a purified enzyme.

12. A method for producing NMN, comprising the step of bringing a transformant with enhanced expression of nicotinamide phosphoribosyltransferase (Nampt) or a treated product thereof into contact with at least nicotinamide (NAM), wherein the transformant has disruption or deletion of a gene encoding an enzyme classified into EC number represented by (d) EC 3.5.1.42, and a gene encoding an enzyme classified into at least any one of EC numbers represented by the following (a), (c), (g), (h) and (i) :
(a) EC 3.1.3.5,
(c) EC 2.4.2.1,
(g) EC 3.2.2.1,
(h) EC 3.2.2.3, and
(i) EC 3.2.2.14.

13. The method for producing NMN according to any one of claims 1 to 12, wherein the total sum of the respective numbers of moles of ATP, ADP and AMP to be added to the reaction system is 0.5 equivalents or less of the number of moles of NMN to be generated.

14. A method for producing nicotinamide mononucleotide (NMN), comprising the step of bringing a transformant with enhanced expression of two enzymes nicotinamide phosphoribosyltransferase (Nampt) and phosphoribosyl pyrophosphate synthetase (Prs), a cell-free protein synthesis reaction solution having the two enzymes expressed, or a treated product thereof into contact with ribose-5-phosphate (R5P), nicotinamide (NAM) and ATP, wherein the total sum of the respective numbers of moles of ATP, ADP and AMP to be added to the reaction system is 0.5 equivalents or less of the number of moles of NMN to be generated.

15. The method for producing NMN according to claim 14, further comprising the step of bringing a transformant with enhanced expression of ribokinase (Rbk), a cell-free protein synthesis reaction solution having the enzyme expressed, or a treated product thereof into contact with ribose and ATP to produce the R5P.


SEQUENCE LISTING

<110>  Mitsubishi Chemical Corporation

<120>  Method for producing nicotinamide mononucleotide and transformant
        for use in the method

<130>  PMC-9019WO

<150>  JP 2017-190028
<151>  2017-09-29

<160>  58

<170>  PatentIn version 3.5

<210>  1
<211>  495
<212>  PRT
<213>  Haemophilus ducreyi

<400>  1

Met Asp Asn Leu Leu Asn Tyr Ser Ser Arg Ala Ser Ala Ile Pro Ser
1               5                   10                  15

Leu Leu Cys Asp Phe Tyr Lys Thr Ser His Arg Ile Met Tyr Pro Glu
            20                  25                  30

Cys Ser Gln Ile Ile Tyr Ser Thr Phe Thr Pro Arg Ser Asn Glu Gln
        35                  40                  45

Ala Pro Tyr Leu Thr Gln Val Val Ser Phe Gly Phe Gln Ala Phe Ile
    50                  55                  60

Ile Lys Tyr Leu Ile His Tyr Phe Asn Asp Asn Phe Phe Ser Arg Asp
65                  70                  75                  80

Lys His Asp Val Val Thr Glu Tyr Ser Ala Phe Ile Glu Lys Thr Leu
                85                  90                  95

Gln Leu Glu Asp Thr Gly Glu His Ile Ala Lys Leu His Glu Leu Gly
            100                 105                 110

Tyr Leu Pro Ile Arg Ile Lys Ala Ile Pro Glu Gly Lys Thr Val Ala
        115                 120                 125

Ile Lys Val Pro Val Met Thr Ile Glu Asn Thr His Ser Asp Phe Phe
    130                 135                 140

Trp Leu Thr Asn Tyr Leu Glu Thr Leu Ile Asn Val Ser Leu Trp Gln
145                 150                 155                 160

Pro Met Thr Ser Ala Ser Ile Ala Phe Ala Tyr Arg Thr Ala Leu Ile
                165                 170                 175

Lys Phe Ala Asn Glu Thr Cys Asp Asn Gln Glu His Val Pro Phe Gln
            180                 185                 190

Ser His Asp Phe Ser Met Arg Gly Met Ser Ser Leu Glu Ser Ala Glu
        195                 200                 205

Thr Ser Gly Ala Gly His Leu Thr Ser Phe Leu Gly Thr Asp Thr Ile
    210                 215                 220

Pro Ala Leu Ser Phe Val Glu Ala Tyr Tyr Gly Ser Ser Ser Leu Ile
225                 230                 235                 240

Gly Thr Ser Ile Pro Ala Ser Glu His Ser Val Met Ser Ser His Gly
                245                 250                 255

Val Asp Glu Leu Ser Thr Phe Arg Tyr Leu Met Ala Lys Phe Pro His
            260                 265                 270

Asn Met Leu Ser Ile Val Ser Asp Thr Thr Asp Phe Trp His Asn Ile
        275                 280                 285

Thr Val Asn Leu Pro Leu Leu Lys Gln Glu Ile Ile Ala Arg Pro Glu
    290                 295                 300

Asn Ala Arg Leu Val Ile Arg Pro Asp Ser Gly Asn Phe Phe Ala Ile
305                 310                 315                 320

Ile Cys Gly Asp Pro Thr Ala Asp Thr Glu His Glu Arg Lys Gly Leu
                325                 330                 335

Ile Glu Cys Leu Trp Asp Ile Phe Gly Gly Thr Val Asn Gln Lys Gly
            340                 345                 350

Tyr Lys Val Ile Asn Pro His Ile Gly Ala Ile Tyr Gly Asp Gly Val
        355                 360                 365

Thr Tyr Glu Lys Met Phe Lys Ile Leu Glu Gly Leu Gln Ala Lys Gly
    370                 375                 380

Phe Ala Ser Ser Asn Ile Val Phe Gly Val Gly Ala Gln Thr Tyr Gln
385                 390                 395                 400

Arg Asn Thr Arg Asp Thr Leu Gly Phe Ala Leu Lys Ala Thr Ser Ile
                405                 410                 415

Thr Ile Asn Gly Glu Glu Lys Ala Ile Phe Lys Asn Pro Lys Thr Asp
            420                 425                 430

Asp Gly Phe Lys Lys Ser Gln Lys Gly Arg Val Lys Val Leu Ser Arg
        435                 440                 445

Asp Thr Tyr Val Asp Gly Leu Thr Ser Ala Asp Asp Phe Ser Asp Asp
    450                 455                 460

Leu Leu Glu Leu Leu Phe Glu Asp Gly Lys Leu Leu Arg Gln Thr Asp
465                 470                 475                 480

Phe Asp Glu Ile Arg Gln Asn Leu Leu Val Ser Arg Thr Thr Leu
                485                 490                 495

<210>  2
<211>  1488
<212>  DNA
<213>  Haemophilus ducreyi

<400>  2
atggacaatc tgctgaacta ctcctcacgc gcctcggcaa tcccgtccct gctgtgcgac      60

ttctacaaaa cctctcatcg tattatgtat ccggaatgct ctcagattat ctatagtacc     120

tttacgccgc gttcgaacga acaggcaccg tacctgaccc aagtggttag cttcggcttc     180

caagctttca tcatcaaata cctgatccat tacttcaacg ataacttttt cagccgtgat     240

aaacacgacg tcgtgaccga atatagtgcg tttattgaaa aaaccctgca gctggaagac      300

acgggcgaac atatcgccaa actgcacgaa ctgggttacc tgccgattcg tatcaaagca     360

attccggaag gtaaaaccgt tgctattaaa gttccggtca tgaccatcga aaatacgcat     420

tctgatttct tttggctgac caactatctg gaaacgctga tcaatgtcag tctgtggcag     480

ccgatgacca gcgcgtcaat tgcgtttgcc taccgtaccg cactgatcaa attcgctaac     540

gaaacgtgtg ataatcagga acatgtgccg tttcaatccc acgacttctc aatgcgcggc     600

atgagctctc tggaatcggc ggaaaccagc ggcgccggtc atctgacgtc ctttctgggt     660

accgatacga ttccggcgct gtcattcgtt gaagcctatt acggcagttc ctcactgatt     720

ggtaccagca tcccggcatc ggaacatagc gttatgtcga gccacggcgt cgatgaactg     780

agcacgtttc gctatctgat ggctaaattc ccgcataaca tgctgtctat tgtcagtgat     840

accacggact tttggcacaa catcaccgtg aatctgccgc tgctgaaaca ggaaattatc     900

gcacgtccgg aaaacgctcg tctggtgatt cgcccggata gcggcaactt tttcgcgatt     960

atctgcggtg atccgaccgc cgacacggaa catgaacgca aaggtctgat tgaatgtctg    1020

tgggatatct ttggcggtac cgtgaaccag aaaggctata aagttatcaa tccgcacatt    1080

ggtgcaatct atggcgacgg tgttacgtac gaaaaaatgt tcaaaatcct ggaaggcctg 1140

caagcgaaag gttttgcctc tagtaacatc gtcttcggcg tgggtgcgca gacctaccaa 1200

cgtaataccc gcgatacgct gggctttgca ctgaaagcta ccagcattac gatcaacggt 1260

gaagaaaaag ccatcttcaa aaacccgaaa accgatgacg gcttcaaaaa atcgcagaaa 1320

ggtcgtgtga aagttctgag ccgcgatacc tatgtggacg gcctgacgtc cgccgatgac 1380

ttttcagatg acctgctgga actgctgttc gaagatggta aactgctgcg tcagaccgac 1440

tttgatgaaa tccgtcagaa cctgctggtt agccgcacga ccctgtga 1488

<210> 3
<211> 317
<212> PRT
<213> Bacillus subtilis

<400> 3

Met Ser Asn Gln Tyr Gly Asp Lys Asn Leu Lys Ile Phe Ser Leu Asn
1               5                   10                  15

Ser Asn Pro Glu Leu Ala Lys Glu Ile Ala Asp Ile Val Gly Val Gln
            20                  25                  30

Leu Gly Lys Cys Ser Val Thr Arg Phe Ser Asp Gly Glu Val Gln Ile
        35                  40                  45

Asn Ile Glu Glu Ser Ile Arg Gly Cys Asp Cys Tyr Ile Ile Gln Ser
    50                  55                  60

Thr Ser Asp Pro Val Asn Glu His Ile Met Glu Leu Leu Ile Met Val
65                  70                  75                  80

Asp Ala Leu Lys Arg Ala Ser Ala Lys Thr Ile Asn Ile Val Ile Pro
                85                  90                  95

Tyr Tyr Gly Tyr Ala Arg Gln Asp Arg Lys Ala Arg Ser Arg Glu Pro
            100                 105                 110

Ile Thr Ala Lys Leu Phe Ala Asn Leu Leu Glu Thr Ala Gly Ala Thr
        115                 120                 125

Arg Val Ile Ala Leu Asp Leu His Ala Pro Gln Ile Gln Gly Phe Phe
    130                 135                 140

Asp Ile Pro Ile Asp His Leu Met Gly Val Pro Ile Leu Gly Glu Tyr
145                 150                 155                 160

Phe Glu Gly Lys Asn Leu Glu Asp Ile Val Ile Val Ser Pro Asp His

                165                 170                 175

Gly Gly Val Thr Arg Ala Arg Lys Leu Ala Asp Arg Leu Lys Ala Pro
            180                 185                 190

Ile Ala Ile Ile Asp Lys Arg Arg Pro Arg Pro Asn Val Ala Glu Val
        195                 200                 205

Met Asn Ile Val Gly Asn Ile Glu Gly Lys Thr Ala Ile Leu Ile Asp
    210                 215                 220

Asp Ile Ile Asp Thr Ala Gly Thr Ile Thr Leu Ala Ala Asn Ala Leu
225                 230                 235                 240

Val Glu Asn Gly Ala Lys Glu Val Tyr Ala Cys Cys Thr His Pro Val
                245                 250                 255

Leu Ser Gly Pro Ala Val Glu Arg Ile Asn Asn Ser Thr Ile Lys Glu
            260                 265                 270

Leu Val Val Thr Asn Ser Ile Lys Leu Pro Glu Glu Lys Lys Ile Glu
        275                 280                 285

Arg Phe Lys Gln Leu Ser Val Gly Pro Leu Leu Ala Glu Ala Ile Ile
    290                 295                 300

Arg Val His Glu Gln Gln Ser Val Ser Tyr Leu Phe Ser
305                 310                 315

<210> 4
<211> 954
<212> DNA
<213> Bacillus subtilis

<400> 4
atgtccaacc aatacggtga caaaaatctg aaaatcttta gcctgaactc caacccggaa 60

ctggcaaaag aaatcgccga catcgtgggc gtgcagctgg gtaaatgcag cgttacccgt 120

tttagcgatg gcgaagtgca gattaacatc gaagaaagca tccgcggttg cgattgttat 180

attatccaga gcaccagcga tccggtgaat gaacatatca tggaactgct gattatggtt 240

gatgcgctga aacgtgcgag cgccaaaacc atcaacatcg ttatcccgta ctacggctat 300

gcgcgtcagg atcgcaaagc ccgtagccgc gaaccgatta ccgcgaaact gtttgccaat 360

ctgctggaaa ccgccggtgc aacccgcgtg attgccctgg atctgcatgc cccgcagatt 420

cagggctttt tcgatatccc gattgatcat ctgatgggcg ttccgatcct gggtgaatat 480

tttgaaggca aaaacctgga agatatcgtg attgttagcc cggatcatgg cggtgtgacc 540

cgtgcgcgta aactggcaga tcgtctgaaa gcgccgattg ccattatcga taaacgtcgc 600

ccgcgcccga atgtggcgga agttatgaac atcgttggca atattgaagg taaaaccgcc 660

atcctgattg atgatattat cgataccgcg ggtaccatta ccctggcggc caacgccctg 720

gtggaaaatg gcgcgaaaga agtttatgcc tgctgtaccc acccggtgct gagcggtccg 780

gccgttgaac gtatcaacaa tagcaccatt aaagaactgg tggttaccaa cagcatcaaa 840

ctgccggaag aaaagaaaat tgaacgcttt aaacagctga gcgtgggccc gctgctggca 900

gaagcgatta ttcgtgtcca tgaacaacaa agcgtcagct acctgttttc ctaa 954

<210> 5
<211> 318
<212> PRT
<213> Homo sapiens

<400> 5

<210>  6
<211>  957
<212>  DNA
<213>  Homo sapiens

<400>  6
atgccgaaca tcaagatttt ctcaggctcg tctcatcagg acctgtcgca aaagatcgcg      60

gaccgtctgg gtctggaact gggcaaggtg gttaccaaaa agtttagcaa ccaggaaacg     120

tgcgtcgaaa ttggcgaatc tgtgcgtggt gaagatgttt atattgtcca aagtggctgt     180

ggtgaaatca acgataatct gatggaactg ctgattatga tcaatgcctg caaaattgca     240

agcgcttctc gcgtgaccgc cgttatcccg tgtttcccgt acgcacgtca ggataaaaag     300

gacaaatcac gcgcaccgat ttcggctaag ctggttgcga acatgctgtc cgtcgcgggc     360

gccgatcata ttatcacgat ggatctgcac gcgtctcaga ttcaaggctt tttcgatatc     420

ccggtggata atctgtatgc agaaccggct gttctgaaat ggattcgtga aaacatctca     480

gaatggcgca attgcaccat tgtgtcgccg gatgcgggcg gtgccaaacg tgtgacgagc     540

attgctgatc gcctgaacgt tgactttgcg ctgatccata aggaacgtaa aaaggcgaat 600

gaagttgatc gcatggtcct ggtgggcgat gtcaaagacc gcgtggctat tctggttgat 660

gacatggcgg atacctgcgg cacgatctgt catgcggccg acaaactgct gagtgcaggt 720

gctacccgtg tgtatgccat tctgacgcac ggcatcttta gcggtccggc aatttctcgc 780

atcaacaatg cctgcttcga agcagtcgtg gttaccaaca cgattccgca ggaagataaa 840

atgaagcact gtagtaaaat ccaagtgatt gacatctcca tgattctggc ggaagccatc 900

cgtcgcaccc ataatggtga aagtgtgtcc tacctgttca gccacgttcc gctgtaa 957

<210> 7
<211> 333
<212> PRT
<213> Saccharomyces cerevisiae

<400> 7

Met Gly Ile Thr Val Ile Gly Ser Leu Asn Tyr Asp Leu Asp Thr Phe
1               5                   10                  15

Thr Asp Arg Leu Pro Asn Ala Gly Glu Thr Phe Arg Ala Asn His Phe
            20                  25                  30

Glu Thr His Ala Gly Gly Lys Gly Leu Asn Gln Ala Ala Ala Ile Gly
        35                  40                  45

Lys Leu Lys Asn Pro Ser Ser Arg Tyr Ser Val Arg Met Ile Gly Asn
    50                  55                  60

Val Gly Asn Asp Thr Phe Gly Lys Gln Leu Lys Asp Thr Leu Ser Asp
65                  70                  75                  80

Cys Gly Val Asp Ile Thr His Val Gly Thr Tyr Glu Gly Ile Asn Thr
                85                  90                  95

Gly Thr Ala Thr Ile Leu Ile Glu Glu Lys Ala Gly Gly Gln Asn Arg
            100                 105                 110

Ile Leu Ile Val Glu Gly Ala Asn Ser Lys Thr Ile Tyr Asp Pro Lys
        115                 120                 125

Gln Leu Cys Glu Ile Phe Pro Glu Gly Lys Glu Glu Glu Glu Tyr Val
    130                 135                 140

Val Phe Gln His Glu Ile Pro Asp Pro Leu Ser Ile Ile Lys Trp Ile
145                 150                 155                 160

His Ala Asn Arg Pro Asn Phe Gln Ile Val Tyr Asn Pro Ser Pro Phe

165                 170                 175

Lys Ala Met Pro Lys Lys Asp Trp Glu Leu Val Asp Leu Leu Val Val
            180                 185                 190

Asn Glu Ile Glu Gly Leu Gln Ile Val Glu Ser Val Phe Asp Asn Glu
        195                 200                 205

Leu Val Glu Glu Ile Arg Glu Lys Ile Lys Asp Asp Phe Leu Gly Glu
    210                 215                 220

Tyr Arg Lys Ile Cys Glu Leu Leu Tyr Glu Lys Leu Met Asn Arg Lys
225                 230                 235                 240

Lys Arg Gly Ile Val Val Met Thr Leu Gly Ser Arg Gly Val Leu Phe
                245                 250                 255

Cys Ser His Glu Ser Pro Glu Val Gln Phe Leu Pro Ala Ile Gln Asn
            260                 265                 270

Val Ser Val Val Asp Thr Thr Gly Ala Gly Asp Thr Phe Leu Gly Gly
        275                 280                 285

Leu Val Thr Gln Leu Tyr Gln Gly Glu Thr Leu Ser Thr Ala Ile Lys
    290                 295                 300

Phe Ser Thr Leu Ala Ser Ser Leu Thr Ile Gln Arg Lys Gly Ala Ala
305                 310                 315                 320

Glu Ser Met Pro Leu Tyr Lys Asp Val Gln Lys Asp Ala
                325                 330

<210>  8
<211>  1002
<212>  DNA
<213>  Saccharomyces cerevisiae

<400>  8
atgggcatta ccgtcattgg ctcgctgaac tacgacctgg atacctttac cgaccgcctg      60

ccgaacgctg gtgaaacctt ccgtgcgaac cattttgaaa cccatgccgg cggtaaaggt     120

ctgaaccagg cggccgcgat tggcaaactg aaaaatccga gcagccgtta tagcgtgcgc     180

atgatcggca acgttggtaa tgataccttt ggtaaacagc tgaaagatac cctgagcgat     240

tgcggcgtgg atattaccca tgttggcacc tatgaaggta tcaataccgg caccgccacc     300

attctgatcg aagaaaaagc gggcggtcag aaccgtattc tgatcgtgga aggtgccaat     360

agcaaaacca tttatgatcc gaaacagctg tgtgaaatct ttccggaagg caaagaagaa     420

gaagaatatg tggtttttca gcatgaaatt ccggacccgc tgagcattat caaatggatt 480

catgcgaacc gcccgaattt tcagatcgtg tataacccga gcccgtttaa aaccatgccg 540

aagaaagatt gggaactggt ggatctgctg gttgtgaacg aaattgaagg tctgcagatc 600

gtggaaagcg tttttgataa tgaactggtg gaagaaatcc gtgaaaaaat caaagatgat 660

tttctgggtg aatatcgcaa aatttgcgaa ctgctgtatg aaaaactgat gaaccgtaaa 720

aaacgcggca tcgtggttat gaccctgggc agccgtggtg tgctgttttg tagccatgaa 780

agcccggaag tgcagtttct gccggccatt cagaatgtta gcgtggttga taccaccggc 840

gcgggtgata cctttctggg cggtctggtt acccagctgt atcagggcga aaccctgagc 900

atggccatta aatttagcac cctggcgagc agcctgacca tccaacgcaa aggtgccgcc 960

gaaagtatgc cgctgtataa agatgttcag aaagatgctt ga 1002

<210> 9
<211> 266
<212> PRT
<213> Deinococcus radiodurans

<400> 9

Met Asp Ile Asp Asn Tyr Arg Val Lys Pro Gly Lys Arg Val Lys Leu
1               5                   10                  15

Ser Asp Trp Ala Thr Asn Asp Asp Ala Gly Leu Ser Lys Glu Glu Gly
            20                  25                  30

Gln Ala Gln Thr Ala Lys Leu Ala Gly Glu Leu Ala Glu Trp Gln Glu
        35                  40                  45

Arg Leu Tyr Ala Glu Gly Lys Gln Ser Leu Leu Leu Ile Leu Gln Ala
    50                  55                  60

Arg Asp Ala Ala Gly Lys Asp Gly Ala Val Lys Lys Val Ile Gly Ala
65                  70                  75                  80

Phe Asn Pro Ala Gly Val Gln Ile Thr Ser Phe Lys Gln Pro Ser Ala
                85                  90                  95

Glu Glu Leu Ser His Asp Phe Leu Trp Arg Ile His Gln Lys Ala Pro
            100                 105                 110

Ala Lys Gly Tyr Val Gly Val Phe Asn Arg Ser Gln Tyr Glu Asp Val
        115                 120                 125

Leu Val Thr Arg Val Tyr Asp Met Ile Asp Asp Lys Thr Ala Lys Arg
    130                 135                 140

Arg Leu Glu His Ile Arg His Phe Glu Glu Leu Leu Thr Asp Asn Ala
145                 150                 155                 160

Thr Arg Ile Val Lys Val Tyr Leu His Ile Ser Pro Glu Glu Gln Lys
                165                 170                 175

Glu Arg Leu Gln Ala Arg Leu Asp Asn Pro Gly Lys His Trp Lys Phe
            180                 185                 190

Asn Pro Gly Asp Leu Lys Asp Arg Ser Asn Trp Asp Lys Phe Asn Asp
        195                 200                 205

Val Tyr Glu Asp Ala Leu Thr Thr Ser Thr Asp Asp Ala Pro Trp Tyr
    210                 215                 220

Val Val Pro Ala Asp Arg Lys Trp Tyr Arg Asp Leu Val Leu Ser His
225                 230                 235                 240

Ile Leu Leu Gly Ala Leu Lys Asp Met Asn Pro Gln Phe Pro Ala Ile
                245                 250                 255

Asp Tyr Asp Pro Ser Lys Val Val Ile His
            260                 265

<210>  10
<211>  801
<212>  DNA
<213>  Deinococcus radiodurans

<400>  10
atggatattg ataactaccg tgtgaaaccg ggcaaacgtg tgaaactgag cgactgggca      60

acgaacgatg atgcgggtct gtccaaagaa gagggtcagg cccagaccgc aaaactggcc     120

ggtgaactgg ccgaatggca ggaacgtctg tatgcggaag gcaaacagag cctgctgctg     180

attctgcagg cccgcgatgc cgccggtaaa gatggtgcgg tgaagaaagt gattggcgcc     240

tttaacccgg cgggtgtgca gatcaccagc tttaaacagc cgagcgcgga agaactgagc     300

catgattttc tgtggcgtat ccatcagaaa gcgccggcca aaggctatgt gggtgttttt     360

aaccgtagcc agtatgaaga tgtgctggtt acccgcgttt atgatatgat tgatgataaa     420

accgccaaac gtcgcctgga acatatccgt cattttgaag aactgctgac cgataatgcg     480

acccgcattg tgaaagttta tctgcatatc agcccggaag aacagaaaga acgtctgcag     540

gcacgtctgg ataacccggg taaacattgg aaatttaatc cgggtgatct gaaagatcgc     600

agcaactggg ataaattcaa cgatgtgtac gaagatgcgc tgaccaccag caccgatgat     660

gccccgtggt atgtggttcc ggcggatcgt aaatggtatc gcgatctggt tctgagccat     720

attctgctgg gtgccctgaa agatatgaat ccgcagtttc cggctattga ctatgacccg     780

tccaaagtgg tgatccattg a                                               801

<210>  11
<211>  317
<212>  PRT
<213>  Bacillus subtilis

<400>  11

Met Ser Asn Gln Tyr Gly Asp Lys Asn Leu Lys Ile Phe Ser Leu Asn
1               5                   10                  15

Ser Asn Pro Glu Leu Ala Lys Glu Ile Ala Asp Ile Val Gly Val Gln
            20                  25                  30

Leu Gly Lys Cys Ser Val Thr Arg Phe Ser Asp Gly Glu Val Gln Ile
        35                  40                  45

Asn Ile Glu Glu Ser Ile Arg Gly Cys Asp Cys Tyr Ile Ile Gln Ser
    50                  55                  60

Thr Ser Asp Pro Val Asn Glu His Ile Met Glu Leu Leu Ile Met Val
65                  70                  75                  80

Asp Ala Leu Lys Arg Ala Ser Ala Lys Thr Ile Asn Ile Val Ile Pro
                85                  90                  95

Tyr Tyr Gly Tyr Ala Arg Gln Asp Arg Lys Ala Arg Ser Arg Glu Pro
            100                 105                 110

Ile Thr Ala Lys Leu Phe Ala Ser Leu Leu Glu Thr Ala Gly Ala Thr
        115                 120                 125

Arg Val Ile Ala Leu Asp Leu His Ala Pro Gln Ile Gln Gly Phe Phe
    130                 135                 140

Asp Ile Pro Ile Asp His Leu Met Gly Val Pro Ile Leu Gly Glu Tyr
145                 150                 155                 160

Phe Glu Gly Lys Asn Leu Glu Asp Ile Val Ile Val Ser Pro Asp His
                165                 170                 175

Gly Gly Val Thr Arg Ala Arg Lys Leu Ala Asp Arg Leu Lys Ala Pro
            180                 185                 190

Ile Ala Ile Ile Asp Lys Arg Arg Pro Arg Pro Asn Val Ala Glu Val
        195                 200                 205

Met Asn Ile Val Gly Asn Ile Glu Gly Lys Thr Ala Ile Leu Ile Asp
    210                 215                 220

Asp Ile Ile Asp Thr Ala Gly Thr Ile Thr Leu Ala Ala Asn Ala Leu
225                 230                 235                 240

Val Glu Asn Gly Ala Lys Glu Val Tyr Ala Cys Cys Thr His Pro Val
                245                 250                 255

Leu Ser Gly Pro Ala Val Glu Arg Ile Asn Asn Ser Thr Ile Lys Glu
            260                 265                 270

Leu Val Val Thr Asn Ser Ile Lys Leu Pro Glu Glu Lys Lys Ile Glu
        275                 280                 285

Arg Phe Lys Gln Leu Ser Val Gly Pro Leu Leu Ala Glu Ala Ile Ile
    290                 295                 300

Arg Val His Glu Gln Gln Ser Val Ser Tyr Leu Phe Ser
305                 310                 315

<210>  12
<211>  954
<212>  DNA
<213>  Bacillus subtilis

<400>  12
atgtccaacc aatacggtga caaaaatctg aaaatcttta gcctgaactc caacccggaa      60

ctggcaaaag aaatcgccga catcgtgggc gtgcagctgg gtaaatgcag cgttacccgt     120

tttagcgatg gcgaagtgca gattaacatc gaagaaagca tccgcggttg cgattgttat     180

attatccaga gcaccagcga tccggtgaat gaacatatca tggaactgct gattatggtt     240

gatgcgctga aacgtgcgag cgccaaaacc atcaacatcg ttatcccgta ctacggctat     300

gcgcgtcagg atcgcaaagc ccgtagccgc gaaccgatta ccgcgaaact gtttgcctct     360

ctgctggaaa ccgccggtgc aacccgcgtg attgccctgg atctgcatgc cccgcagatt     420

cagggctttt tcgatatccc gattgatcat ctgatgggcg ttccgatcct gggtgaatat     480

tttgaaggca aaaacctgga agatatcgtg attgttagcc cggatcatgg cggtgtgacc     540

cgtgcgcgta aactggcaga tcgtctgaaa gcgccgattg ccattatcga taaacgtcgc     600

ccgcgcccga atgtggcgga agttatgaac atcgttggca atattgaagg taaaaccgcc     660

atcctgattg atgatattat cgataccgcg ggtaccatta ccctggcggc caacgccctg     720

gtggaaaatg gcgcgaaaga agtttatgcc tgctgtaccc acccggtgct gagcggtccg     780

gccgttgaac gtatcaacaa tagcaccatt aaagaactgg tggttaccaa cagcatcaaa     840

ctgccggaag aaaagaaaat tgaacgcttt aaacagctga gcgtgggccc gctgctggca 900

gaagcgatta ttcgtgtcca tgaacaacaa agcgtcagct acctgttttc ctaa 954

<210> 13
<211> 317
<212> PRT
<213> Bacillus subtilis

<400> 13

Met Ser Asn Gln Tyr Gly Asp Lys Asn Leu Lys Ile Phe Ser Leu Asn
1               5                   10                  15

Ser Asn Pro Glu Leu Ala Lys Glu Ile Ala Asp Ile Val Gly Val Gln
            20                  25                  30

Leu Gly Lys Cys Ser Val Thr Arg Phe Ser Asp Gly Glu Val Gln Ile
        35                  40                  45

Asn Ile Glu Glu Ser Ile Arg Gly Cys Asp Cys Tyr Ile Ile Gln Ser
    50                  55                  60

Thr Ser Asp Pro Val Asn Glu His Ile Met Glu Leu Leu Ile Met Val
65                  70                  75                  80

Asp Ala Leu Lys Arg Ala Ser Ala Lys Thr Ile Asn Ile Val Ile Pro
                85                  90                  95

Tyr Tyr Gly Tyr Ala Arg Gln Asp Arg Lys Ala Arg Ser Arg Glu Pro
            100                 105                 110

Ile Thr Ala Lys Leu Phe Ala Asn Leu Leu Glu Thr Ala Gly Ala Thr
        115                 120                 125

Arg Val Ile Ala Leu Asp Ile His Ala Pro Gln Ile Gln Gly Phe Phe
    130                 135                 140

Asp Ile Pro Ile Asp His Leu Met Gly Val Pro Ile Leu Gly Glu Tyr
145                 150                 155                 160

Phe Glu Gly Lys Asn Leu Glu Asp Ile Val Ile Val Ser Pro Asp His
                165                 170                 175

Gly Gly Val Thr Arg Ala Arg Lys Leu Ala Asp Arg Leu Lys Ala Pro
            180                 185                 190

Ile Ala Ile Ile Asp Lys Arg Arg Pro Arg Pro Asn Val Ala Glu Val
        195                 200                 205

Met Asn Ile Val Gly Asn Ile Glu Gly Lys Thr Ala Ile Leu Ile Asp
    210                 215                 220

Asp Ile Ile Asp Thr Ala Gly Thr Ile Thr Leu Ala Ala Asn Ala Leu
225                 230                 235                 240

Val Glu Asn Gly Ala Lys Glu Val Tyr Ala Cys Cys Thr His Pro Val
                245                 250                 255

Leu Ser Gly Pro Ala Val Glu Arg Ile Asn Asn Ser Thr Ile Lys Glu
            260                 265                 270

Leu Val Val Thr Asn Ser Ile Lys Leu Pro Glu Glu Lys Lys Ile Glu
        275                 280                 285

Arg Phe Lys Gln Leu Ser Val Gly Pro Leu Leu Ala Glu Ala Ile Ile
    290                 295                 300

Arg Val His Glu Gln Gln Ser Val Ser Tyr Leu Phe Ser
305                 310                 315

<210>  14
<211>  954
<212>  DNA
<213>  Bacillus subtilis

<400>  14
atgtccaacc aatacggtga caaaaatctg aaaatcttta gcctgaactc caacccggaa      60

ctggcaaaag aaatcgccga catcgtgggc gtgcagctgg gtaaatgcag cgttacccgt     120

tttagcgatg gcgaagtgca gattaacatc gaagaaagca tccgcggttg cgattgttat     180

attatccaga gcaccagcga tccggtgaat gaacatatca tggaactgct gattatggtt     240

gatgcgctga aacgtgcgag cgccaaaacc atcaacatcg ttatcccgta ctacggctat     300

gcgcgtcagg atcgcaaagc ccgtagccgc gaaccgatta ccgcgaaact gtttgccaat     360

ctgctggaaa ccgccggtgc aacccgcgtg attgccctgg atatccatgc cccgcagatt     420

cagggctttt tcgatatccc gattgatcat ctgatgggcg ttccgatcct gggtgaatat     480

tttgaaggca aaaacctgga agatatcgtg attgttagcc cggatcatgg cggtgtgacc     540

cgtgcgcgta aactggcaga tcgtctgaaa gcgccgattg ccattatcga taaacgtcgc     600

ccgcgcccga atgtggcgga agttatgaac atcgttggca atattgaagg taaaaccgcc     660

atcctgattg atgatattat cgataccgcg ggtaccatta ccctggcggc caacgccctg     720

gtggaaaatg gcgcgaaaga agtttatgcc tgctgtaccc acccggtgct gagcggtccg     780

gccgttgaac gtatcaacaa tagcaccatt aaagaactgg tggttaccaa cagcatcaaa     840

ctgccggaag aaaagaaaat tgaacgcttt aaacagctga gcgtgggccc gctgctggca 900

gaagcgatta ttcgtgtcca tgaacaacaa agcgtcagct acctgttttc ctaa 954

<210> 15
<211> 27
<212> DNA
<213> Artificial Sequence

<220>
<223> Primer (forward) for mutagenesis of Prs mutant BsPrsN120S

<400> 15
tttgcctctc tgctggaaac cgccggt 27

<210> 16
<211> 27
<212> DNA
<213> Artificial Sequence

<220>
<223> Primer (reverse) for mutagenesis of Prs mutant BsPrsN120S

<400> 16
cagcagagag gcaaacagtt tcgcggt 27

<210> 17
<211> 27
<212> DNA
<213> Artificial Sequence

<220>
<223> Primer (forward) for mutagenesis of Prs mutant BsPrsL135I

<400> 17
ctggatatcc atgccccgca gattcag 27

<210> 18
<211> 27
<212> DNA
<213> Artificial Sequence

<220>
<223> Primer (reverse) for mutagenesis of Prs mutant BsPrsL135I

<400> 18
ggcatggata tccagggcaa tcacgcg 27

<210> 19
<211> 20
<212> DNA
<213> Artificial Sequence

<220>
<223> Primer T7-PP

<400> 19
taatacgact cactataggg 20

<210>  20
<211>  19
<212>  DNA
<213>  Artificial Sequence

<220>
<223>  Primer T7-TP

<400>  20
gctagttatt gctcagcgg                                                    19


<210>  21
<211>  462
<212>  PRT
<213>  Deinococcus radiodurans

<400>  21

Met Thr Thr Pro Leu Ser Asp Leu Asn Leu Ile Leu Asp Thr Asp Ser
1               5                   10                  15

Tyr Lys Ser Ser His Phe Leu Gln Tyr Pro Pro Gly Thr Thr Arg Leu
            20                  25                  30

Phe Ser Tyr Leu Glu Ser Arg Gly Gly Arg Tyr Pro Val Thr Arg Phe
        35                  40                  45

Phe Gly Leu Gln Tyr Ile Leu Ser Arg Tyr Leu Thr Arg Arg Val Thr
    50                  55                  60

Met Glu Met Val Glu Glu Ala Arg Ala Val Ile Glu Ala His Gly Glu
65                  70                  75                  80

Pro Phe Pro Tyr Glu Gly Trp Arg Arg Val Val Glu Val His Gly Gly
                85                  90                  95

Lys Leu Pro Leu Glu Ile Arg Ala Val Pro Glu Gly Thr Leu Val Pro
            100                 105                 110

Ile His Asn Val Leu Met Ser Cys Thr Asn Thr Asp Pro Glu Leu Pro
        115                 120                 125

Trp Leu Pro Gly Trp Phe Glu Thr Met Leu Met Arg Val Trp Tyr Pro
    130                 135                 140

Thr Thr Val Cys Thr Gln Ser Trp His Ile Arg Glu Ile Ile Arg Gln
145                 150                 155                 160

Ala Leu Glu Asp Thr Ser Asp Arg Ala Ala Glu Glu Leu Pro Phe Lys
                165                 170                 175

Leu His Asp Phe Gly Ser Arg Gly Val Ser Ser Arg Glu Ser Ala Gly
            180                 185                 190

Ile Gly Gly Leu Ala His Leu Val Asn Phe Gln Gly Ser Asp Thr Leu
        195                 200                 205

Glu Ala Leu Arg Val Gly Arg Asn Tyr Tyr Gly Ala Glu Leu Ala Gly
    210                 215                 220

Phe Ser Ile Pro Ala Ala Glu His Ser Thr Ile Thr Ser Trp Gly Lys
225                 230                 235                 240

Glu His Glu Val Asp Ala Tyr Arg Asn Met Val Arg Gln Phe Gly Lys
                245                 250                 255

Pro Gly Lys Val Tyr Ala Val Val Ser Asp Ser Tyr Asp Leu Lys His
            260                 265                 270

Ala Ile Asn Val His Trp Gly Glu Thr Leu Arg Lys Glu Val Glu Glu
        275                 280                 285

Ser Gly Gly Thr Leu Val Val Arg Pro Asp Ser Gly Asp Pro Pro Ala
    290                 295                 300

Met Val Arg Leu Ala Val Asn Ala Leu Ala Ala Lys Tyr Gly Thr Thr
305                 310                 315                 320

Thr Asn Ser Lys Gly Phe Lys Val Leu Gln His Val Arg Val Ile Gln
                325                 330                 335

Gly Asp Gly Ile Asp Glu Ser Thr Ile Arg Gln Ile Leu Gln Asn Leu
            340                 345                 350

Tyr Val Asp Gly Phe Ser Ala Glu Asn Val Thr Phe Gly Met Gly Gly
        355                 360                 365

Ala Leu Leu Gln Lys Val Asp Arg Asp Thr Gln Arg Phe Ala Tyr Lys
    370                 375                 380

Ala Ser Ala Gly Leu Ile Asp Gly Glu Tyr Arg Gly Ile Tyr Lys Asp
385                 390                 395                 400

Pro Val Thr Asp Pro Gly Lys Arg Ser Lys Asp Gly Val Leu Asp Leu
                405                 410                 415

Val Glu Glu Asn Gly Arg Met Val Thr Arg Gln Tyr Arg Thr Phe Asp

420                 425                 430

Thr Asp Phe Pro Gly Ser Leu Met Arg Thr Val Tyr Arg Asp Gly Glu
        435                 440                 445

Leu Leu Val Gln Asp Thr Leu Glu Glu Ile Arg Gly Arg Gly
    450                 455                 460

<210>  22
<211>  1389
<212>  DNA
<213>  Deinococcus radiodurans

<400>  22
atgacgacgc cgctgtccga cctgaacctg attctggata ccgactccta caaaagctcg      60

catttcctgc aatacccgcc gggcaccacc cgtctgttta gctatctgga aagccgtggc     120

ggtcgctatc cggtgacccg tttctttggc ctgcagtata ttctgagccg ctatctgacc     180

cgtcgcgtga cgatggaaat ggttgaagaa gcgcgtgccg ttatcgaagc gcatggcgaa     240

ccgtttccgt atgaaggttg gcgtcgcgtg gttgaagtgc atggcggtaa actgccgctg     300

gaaattcgtg cggttccgga aggtaccctg gtgccgatcc ataacgttct gatgagctgc     360

accaataccg atccggaact gccgtggctg ccgggttggt ttgaaaccat gctgatgcgt     420

gtgtggtatc cgaccaccgt ttgtacccag agctggcata ttcgtgaaat tatccgccag     480

gcgctggaag ataccagcga tcgcgcggcc gaagaactgc cgtttaaact gcatgatttt     540

ggcagccgtg gtgtgagcag ccgcgaaagc gccggtatcg gcggtctggc acatctggtg     600

aactttcagg gtagcgatac cctggaagcg ctgcgtgttg gtcgcaatta ttatggcgcg     660

gaactggccg gttttagcat tccggcggcc gaacatagca ccatcaccag ctggggtaaa     720

gaacatgaag tggatgccta tcgtaacatg gttcgccagt ttggcaaacc gggtaaagtg     780

tatgcggtgg ttagcgatag ctatgatctg aaacatgcca ttaatgtgca ttggggcgaa     840

accctgcgca aagaagttga agaaagcggc ggtaccctgg tggttcgtcc ggatagcggt     900

gatccgccgg cgatggtgcg tctggccgtt aatgcactgg ccgccaaata tggcaccacc     960

accaatagca aaggttttaa agtgctgcag catgtgcgtg ttattcaggg cgatggtatc    1020

gatgaagcca ccattcgcca gatcctgcag aacctgtatg tggatggctt tagcgcggaa    1080

aatgttacct ttggtatggg cggtgccctg ctgcagaaag tggatcgtga tacccagcgc    1140

tttgcctata aagcgagcgc cggcctgatt gatggtgaat atcgtggcat ctataaagat    1200

ccggttaccg atccgggcaa acgcagcaaa gatggtgtgc tggatctggt tgaagaaaac    1260

ggccgtatgg tgacccgtca gtatcgcacc tttgataccg attttccggg tagcctgatg    1320

cgcaccgttt accgtgatgg tgaactgctg gtgcaagata ccctggaaga aatccgtggt    1380

cgtggctga                                                              1389

<210>  23
<211>  490
<212>  PRT
<213>  Shewanella oneidensis

<400>  23

Met Tyr Leu Asn Pro Val Thr Ala Ile Asp Gly Tyr Lys Val Asp His
1               5                   10                  15

Arg Arg Gln Tyr Pro Asp Asn Thr Gln Val Ile Phe Ser Asn Leu Thr
            20                  25                  30

Ala Arg Lys Ser Arg Arg Gly Tyr Thr Asp Gln Met Val Phe Phe Gly
        35                  40                  45

Leu Gln Tyr Phe Ile Lys His Tyr Leu Ile Asp Ser Trp Asn Arg Asp
    50                  55                  60

Phe Phe Gln Gln Pro Lys Glu Gln Val Ile Cys Gln Phe Ser Arg Arg
65                  70                  75                  80

Ile Asn Asn Tyr Leu Gly Pro Asn Asn Val Gly Thr Gln His Ile Glu
                85                  90                  95

Glu Leu His Asp Leu Gly Tyr Leu Pro Ile Lys Ile Met Ala Leu Pro
            100                 105                 110

Glu Gly Ser Val Tyr Pro Leu Lys Val Pro Cys Leu Ile Leu Tyr Asn
        115                 120                 125

Thr Asp Glu Arg Phe Phe Trp Leu Thr Asn Tyr Leu Glu Thr Ile Leu
    130                 135                 140

Ser Ala Asn Val Trp Gly Met Cys Thr Ser Ala Thr Thr Ala Leu Gln
145                 150                 155                 160

Tyr Arg Lys Ile Phe Glu Ala Tyr Ala Leu Glu Thr Asp Gly Asp Ile
                165                 170                 175

Ala Phe Val Asp Trp Gln Gly His Asp Phe Ser Phe Arg Gly Met Tyr
            180                 185                 190

Gly Val Glu Ala Ala Ile Met Ser Gly Ala Ala His Leu Leu Ser Phe
        195                 200                 205

Thr Gly Thr Asp Thr Ile Pro Ala Ile Asp Phe Leu Glu Gln Tyr Tyr

210                 215                 220

Leu Ala Asp Ser Asp Lys Glu Leu Val Gly Gly Ser Val Pro Ala Thr
225                 230                 235                 240

Glu His Ser Val Met Cys Ala Gly Gly Met Glu Asn Glu Leu Glu Thr
                245                 250                 255

Phe Arg Arg Leu Ile Glu Asp Ile Tyr Pro Thr Gly Ile Val Ser Ile
            260                 265                 270

Val Ser Asp Ser Trp Asp Phe Trp Gln Val Met Thr Glu Phe Thr Leu
        275                 280                 285

Ala Leu Lys Asp Arg Ile Leu Ala Arg Asp Gly Lys Val Val Phe Arg
    290                 295                 300

Pro Asp Thr Gly Cys Pro Val Lys Ile Ile Cys Gly Asp Pro Gln Ala
305                 310                 315                 320

Pro Ile Gly Ser Pro Glu Tyr Lys Gly Ala Ile Glu Cys Leu Trp Asp
                325                 330                 335

Val Phe Gly Gly Ser Thr Thr Ala Lys Gly Tyr Lys Leu Leu Asp Ser
            340                 345                 350

His Val Gly Leu Ile Tyr Gly Asp Ser Ile Thr Ile Glu Arg Ala Glu
        355                 360                 365

Ala Ile Cys Ala Gly Leu Lys Ala Lys Gly Phe Ala Ser Thr Asn Ile
    370                 375                 380

Val Phe Gly Ile Gly Ser Phe Thr Tyr Gln His Val Thr Arg Asp Thr
385                 390                 395                 400

Asp Gly Tyr Ala Val Lys Ala Thr Phe Ala Lys Val Asp Gly Lys Asp
                405                 410                 415

Arg Glu Ile Phe Lys Asp Pro Lys Thr Asp Asp Gly Thr Lys Lys Ser
            420                 425                 430

Ala Lys Gly Leu Val Ala Val Phe Lys Asp Glu Gln Gly Gln Phe Tyr
        435                 440                 445

Leu Lys Asp Gln Ala Ser Trp Gln Asp Val Asn Asn Cys Glu Phe Val
    450                 455                 460

Pro Val Phe Ala Asp Gly Glu Leu Leu Thr Glu Tyr Ser Leu Ala Asp
465                 470                 475                 480

Ile Arg Ala Arg Leu Ala Ala Ser Arg Arg
                485                 490

<210>  24
<211>  1473
<212>  DNA
<213>  Shewanella oneidensis

<400>  24
atgtatctga acccggtgac ggcaatcgac ggctacaaag ttgaccatcg ccgccaatac      60

ccggacaata cccaagttat cttctcaaac ctgacggcgc gtaaaagccg tcgcggctat     120

accgatcaga tggtgttttt cggtctgcaa tacttcatca aacattacct gatcgatagt     180

tggaatcgcg actttttcca gcaaccgaaa gaacaagtga tttgccaatt ctcccgtcgc     240

atcaacaatt atctgggccc gaacaatgtt ggtacgcagc atattgaaga actgcacgat     300

ctgggctacc tgccgattaa aatcatggcc ctgccggaag gtagcgttta tccgctgaaa     360

gtcccgtgcc tgattctgta caacaccgat gaacgtttct tttggctgac gaactatctg     420

gaaaccatcc tgagcgccaa tgtgtggggc atgtgtacgt ctgcgaccac ggccctgcag     480

tatcgtaaaa tttttgaagc gtacgccctg gaaaccgatg gcgacatcgc attcgttgat     540

tggcaaggtc atgactttag cttccgcggc atgtacggtg tcgaagcggc cattatgagt     600

ggcgcagctc acctgctgtc ctttaccggt acggatacca ttccggcaat cgacttcctg     660

gaacagtatt acctggctga ttctgacaaa gaactggtcg gcggttcagt gccggcgacc     720

gaacactcgg ttatgtgtgc cggcggtatg gaaaatgaac tggaaacgtt tcgtcgcctg     780

attgaagata tctatccgac cggcattgtg agtatcgtta gcgattcttg ggacttttgg     840

caggtgatga cggaattcac cctggcactg aaagatcgta tcctggctcg cgacggtaaa     900

gtggtttttc gtccggatac cggctgcccg gttaaaatta tctgtggtga cccgcaggcc     960

ccgattggta gcccggaata caaaggtgcc atcgaatgcc tgtgggatgt ctttggcggt    1020

tctaccacgg cgaaaggtta taaactgctg gatagtcatg ttggcctgat ttacggtgac    1080

tccattacca tcgaacgcgc agaagctatc tgtgccggcc tgaaagcaaa aggttttgct    1140

tcaacgaaca ttgtctttgg catcggttcg ttcacctatc agcacgtgac gcgtgatacc    1200

gacggctacg cagtgaaagc tacctttgcg aaagttgatg gtaaagaccg cgaaattttc    1260

aaagatccga aaacggatga cggcaccaaa aaatcagcga aaggtctggt cgccgtgttt    1320

aaagatgaac agggccaatt ctatctgaaa gatcaggcat cgtggcaaga cgtgaacaat    1380

tgcgaatttg ttccggtctt cgctgatggt gaactgctga ccgaatactc cctggctgac    1440

atccgtgccc gcctggccgc atcccgtcgc taa                                 1473

<210>  25
<211>  27
<212>  DNA
<213>  Artificial Sequence

<220>
<223>  Primer (forward) for mutagenesis of His-tag added Nampt
       expression plasmid pEHdNampt-His

<400>  25
acgaccctgc accaccacca ccaccac                                          27


<210>  26
<211>  27
<212>  DNA
<213>  Artificial Sequence

<220>
<223>  Primer (reverse) for mutagenesis of His-tag added Nampt
       expression plasmid pEHdNampt-His

<400>  26
gtggtggtgc agggtcgtgc ggctaac                                          27


<210>  27
<211>  27
<212>  DNA
<213>  Artificial Sequence

<220>
<223>  Primer (forward) for mutagenesis of His-tag added Nampt
       expression plasmid pEDrNampt-His

<400>  27
ggtcgtggcc accaccacca ccaccac                                          27


<210>  28
<211>  27
<212>  DNA
<213>  Artificial Sequence

<220>
<223>  Primer (reverse) for mutagenesis of His-tag added Nampt
       expression plasmid pEDrNampt-His

<400>  28
gtggtggtgg ccacgaccac ggatttc                                          27


<210>  29
<211>  27
<212>  DNA
<213>  Artificial Sequence

<220>
<223>  Primer (forward) for mutagenesis of His-tag added Nampt
       expression plasmid pESoNampt-His

<400>  29
tcccgtcgcc accaccacca ccaccac                                          27


<210>  30
<211>  27
<212>  DNA
<213>  Artificial Sequence

<220>
<223>  Primer (reverse) for mutagenesis of His-tag added Nampt
       expression plasmid pESoNampt-His

<400>  30
gtggtggtgg cgacgggatg cggccag                                          27


<210>  31
<211>  53
<212>  DNA
<213>  Artificial Sequence

<220>
<223>  IBS primer for ushA

<400>  31
aaaaaagctt ataattatcc ttaggtagcg tgctggtgcg cccagatagg gtg             53


<210>  32
<211>  60
<212>  DNA
<213>  Artificial Sequence

<220>
<223>  EBS1d primer for ushA

<400>  32
cagattgtac aaatgtggtg ataacagata agtcgtgctg cttaacttac ctttctttgt      60


<210>  33
<211>  49
<212>  DNA
<213>  Artificial Sequence

<220>
<223>  EBS2 primer for ushA

<400>  33
tgaacgcaag tttctaattt cgattctacc tcgatagagg aaagtgtct                  49


<210>  34
<211>  30
<212>  DNA
<213>  Artificial Sequence

<220>
<223>  Primer (forward) for preparation of plasmid pACD4K-C-ushAdKm

<400>  34
ttggcgtata acatagtatc gacggagccg                                       30

<210>  35
<211>  30
<212>  DNA
<213>  Artificial Sequence

<220>
<223>  Primer (reverse) for preparation of plasmid pACD4K-C-ushAdKm

<400>  35
acgcgtcgcc acgtaataaa tatctggacg                                        30


<210>  36
<211>  20
<212>  DNA
<213>  Artificial Sequence

<220>
<223>  Primer (forward) for detection of disruption of ushA gene

<400>  36
atggtatccg caaagaggtt                                                   20


<210>  37
<211>  20
<212>  DNA
<213>  Artificial Sequence

<220>
<223>  Primer (reverse) for detection of disruption of ushA gene

<400>  37
acctctttac cggtcatgtc                                                   20


<210>  38
<211>  53
<212>  DNA
<213>  Artificial Sequence

<220>
<223>  IBS primer for pncA

<400>  38
aaaaaagctt ataattatcc ttaatcgacg aattggtgcg cccagatagg gtg              53


<210>  39
<211>  60
<212>  DNA
<213>  Artificial Sequence

<220>
<223>  EBS1d primer for pncA

<400>  39
cagattgtac aaatgtggtg ataacagata agtcgaattg attaacttac ctttctttgt       60


<210>  40

<211>  49
<212>  DNA
<213>  Artificial Sequence

<220>
<223>  EBS2 primer for pncA

<400>  40
tgaacgcaag tttctaattt cggtttcgat ccgatagagg aaagtgtct                49


<210>  41
<211>  20
<212>  DNA
<213>  Artificial Sequence

<220>
<223>  Primer (forward) for detection of disruption of pncA gene

<400>  41
aattgcccag ggccacgtct                                                20


<210>  42
<211>  27
<212>  DNA
<213>  Artificial Sequence

<220>
<223>  Primer (reverse) for detection of disruption of pncA gene

<400>  42
   ccagcgtaaa attacccctg tgtctct                                       27


<210>  43
<211>  53
<212>  DNA
<213>  Artificial Sequence

<220>
<223>  IBS primer for pncC

<400>  43
aaaaaagctt ataattatcc ttagattacg ccgtggtgcg cccagatagg gtg           53


<210>  44
<211>  60
<212>  DNA
<213>  Artificial Sequence

<220>
<223>  EBS1d primer for pncC

<400>  44
cagattgtac aaatgtggtg ataacagata agtcgccgtg tctaacttac ctttctttgt    60


<210>  45
<211>  49
<212>  DNA
<213>  Artificial Sequence

<220>
<223>  EBS2 primer for pncC

<400>  45
tgaacgcaag tttctaattt cgatttaatc tcgatagagg aaagtgtct          49


<210>  46
<211>  25
<212>  DNA
<213>  Artificial Sequence

<220>
<223>  Primer (forward) for detection of disruption of pncC gene

<400>  46
ctgacagtga actgatgcag ttaag                                    25


<210>  47
<211>  26
<212>  DNA
<213>  Artificial Sequence

<220>
<223>  Primer (reverse) for detection of disruption of pncC gene

<400>  47
tcaagtgttt tgtagaaatt gttgcc                                   26


<210>  48
<211>  53
<212>  DNA
<213>  Artificial Sequence

<220>
<223>  IBS primer for yrfG

<400>  48
aaaaaagctt ataattatcc ttaggcacct tgctcgtgcg cccagatagg gtg     53


<210>  49
<211>  60
<212>  DNA
<213>  Artificial Sequence

<220>
<223>  EBS1d primer for yrfG

<400>  49
cagattgtac aaatgtggtg ataacagata agtcttgctc gataacttac ctttctttgt     60


<210>  50
<211>  49
<212>  DNA
<213>  Artificial Sequence

<220>
<223>  EBS2 primer for yrfG

<400>  50
tgaacgcaag tttctaattt cgattgtgcc tcgatagagg aaagtgtct                    49


<210>  51
<211>  18
<212>  DNA
<213>  Artificial Sequence

<220>
<223>  Primer (forward) for detection of disruption of yrfG gene

<400>  51
gttctgctgg atatggac                                                      18


<210>  52
<211>  18
<212>  DNA
<213>  Artificial Sequence

<220>
<223>  Primer (reverse) for detection of disruption of yrfG gene

<400>  52
aaatgtgtgg gtggaaag                                                      18


<210>  53
<211>  27
<212>  DNA
<213>  Artificial Sequence

<220>
<223>  Primer (forward) for mutagenesis of His-tag added Nampt
       expression plasmid pEBsPrsN120St-His

<400>  53
ctgttttccc accaccacca ccaccac                                            27


<210>  54
<211>  27
<212>  DNA
<213>  Artificial Sequence

<220>
<223>  Primer (reverse) for mutagenesis of His-tag added Nampt
       expression plasmid pEBsPrsN120St-His

<400>  54
gtggtggtgg gaaaacaggt agctgac                                            27


<210>  55
<211>  27
<212>  DNA
<213>  Artificial Sequence

<220>
<223>  Primer (forward) for mutagenesis of His-tag added Nampt
       expression plasmid pEDrPpk-His

<400>  55
gtgatccatc accaccacca ccaccac                                          27


<210>  56
<211>  27
<212>  DNA
<213>  Artificial Sequence

<220>
<223>  Primer (reverse) for mutagenesis of His-tag added Nampt
       expression plasmid pEDrPpk-His

<400>  56
gtggtggtga tggatcacca ctttgga                                          27


<210>  57
<211>  27
<212>  DNA
<213>  Artificial Sequence

<220>
<223>  Primer (forward) for mutagenesis of His-tag added Nampt
       expression plasmid pEScRbk-His

<400>  57
gatgttcaga aagatgctca ccaccac                                          27


<210>  58
<211>  27
<212>  DNA
<213>  Artificial Sequence

<220>
<223>  Primer (reverse) for mutagenesis of His-tag added Nampt
       expression plasmid pEScRbk-His

<400>  58
gtggtggtga gcatctttct gaacatc                                          27